# EUROPEAN PATENT APPLICATION

(11) **EP 2 848 937 A1**
(43) Date of publication of application: **18.03.2015**
(21) Application number: 14003072.7
(22) Date of filing: 05.09.2014
(51) Int. Cl.: G01N 33/543, G01N 33/569

(54) **Methods of identifying novel HIV-1 immunogens**

(30) Priority: 05.09.2013 US 201361874124 P
(71) Applicant: International Aids Vaccine Initiative, New York, NY 10004 (US); Monogram BioSciences, Inc., South San Francisco, CA 94080-1913 (US); Caulfield, Michael, Fort Washington, PA 13034 (US); Hoffenberg, Simon, Hartsdale, NY 10530 (US); King, C. Richter, Washington, DC 20010 (US); Petropoulos, Christos, South San Francisco, CA (US); Phogat, Sanjay, Edison, NJ 08820 (US); Wagner, Denise, Brooklyn, NY 11207 (US); Wrin, Terri, South San Francisco, CA (US)
(72) Inventor: Caulfield, Michael, Fort Washington, PA 13034 (US); Hoffenberg, Simon, Hartsdale, NY 10530 (US); King, Richter C., Washington, DC 20010 (US); Petropoulos, Christos, Half Moon Bay, CA 94019 (US); Phogat, Sanjay, Edison, NJ 08820 (US); Wagner, Denise, Brooklyn, NY 11207 (US); Wrin, Terri, Fremont, CA 94555 (US)
(74) Representative: Schüssler, Andrea

(57) **Abstract**

The present application relates to identifying one or more components of HIV envelope glycoprotein which bind to broadly neutralizing antibodies, which may be utilized as research tools for developing HIV-1 vaccine immunogens, antigens for crystallization and/or for identifying of broad neutralizing antibodies.

## Description

### RELATED APPLICATIONS AND INCORPORATION BY REFERENCE

This application claims benefit of and priority to US provisional patent application Serial No. 61/874,124 filed September 5, 2013.

The foregoing applications, and all documents cited therein or during their prosecution ("appln cited documents") and all documents cited or referenced in the appln cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### FIELD OF THE INVENTION

The present invention relates to methods of identifying novel HIV immunogens, such as envelope (env) sequences encoding such immunogens, which may be useful for generating HIV vaccines or for generating HIV pseudoviruses and/or panels thereof.

### BACKGROUND OF THE INVENTION

AIDS, or Acquired Immunodeficiency Syndrome, is caused by human immunodeficiency virus (HIV) and is characterized by several clinical features including wasting syndromes, central nervous system degeneration and profound immunosuppression that results in opportunistic infections and malignancies. HIV is a member of the lentivirus family of animal retroviruses, which include the visna virus of sheep and the bovine, feline, and simian immunodeficiency viruses (SIV). Two closely related types of HIV, designated HIV-1 and HIV-2, have been identified thus far, of which HIV-1 is by far the most common cause of AIDS. However, HIV-2, which differs in genomic structure and antigenicity, causes a similar clinical syndrome.

An infectious HIV particle consists of two identical strands of RNA, each approximately 9.2 kb long, packaged within a core of viral proteins. This core structure is surrounded by a phospholipid bilayer envelope derived from the host cell membrane that also includes virally-encoded membrane proteins (Abbas et al., Cellular and Molecular Immunology, 4th edition, W.B. Saunders Company, 2000, p. 454). The HIV genome has the characteristic 5'-LTR-Gag-Pol-Env-LTR-3' organization of the retrovirus family. Long terminal repeats (LTRs) at each end of the viral genome serve as binding sites for transcriptional regulatory proteins from the host and regulate viral integration into the host genome, viral gene expression, and viral replication.

The HIV genome encodes several proteins. The *gag* gene encodes structural proteins of the nucleocapsid core and matrix. The *pol* gene encodes reverse transcriptase (RT), integrase (IN), and viral protease (PR) enzymes required for viral replication. The *tat* gene encodes a protein that is required for elongation of viral transcripts. The *rev* gene encodes a protein that promotes the nuclear export of incompletely spliced or unspliced viral RNAs. The *vif* gene product enhances the infectivity of viral particles. The *vpr* gene product promotes the nuclear import of viral DNA and regulates G2 cell cycle arrest. The *vpu* and *nef* genes encode proteins that down regulate host cell CD4 expression and enhance release of virus from infected cells. The *env* gene encodes the viral envelope glycoprotein that is translated as a 160-kilodalton (kDa) precursor (gp160) and cleaved by a cellular protease to yield the external 120-kDa envelope glycoprotein (gp120) and the transmembrane 41-kDa envelope glycoprotein (gp41), which are required for the infection of cells (Abbas et al., Cellular and Molecular Immunology, 4th edition, W.B. Saunders Company, 2000, pp. 454-456). gp140 is a modified form of the Env glycoprotein, which contains the external 120-kDa envelope glycoprotein portion and the extracellular part of the gp41 portion of Env and has characteristics of both gp120 and gp41. The *nef* gene is conserved among primate lentiviruses and is one of the first viral genes that is transcribed following infection. *In vitro,* several functions have been described, including down-regulation of CD4 and MHC class I surface expression, altered T-cell signaling and activation, and enhanced viral infectivity.

HIV infection initiates with gp120 on the viral particle binding to the CD4 and chemokine receptor molecules (e.g., CXCR4, CCR5) on the cell membrane of target cells such as CD4⁺ T-cells, macrophages and dendritic cells. The bound virus fuses with the target cell and reverse transcribes the RNA genome. The resulting viral DNA integrates into the cellular genome, where it directs the production of new viral RNA, and thereby viral proteins and new virions. These virions bud from the infected cell membrane and establish productive infections in other cells. This process also kills the originally infected cell. HIV can also kill cells indirectly because the CD4 receptor on uninfected T-cells has a strong affinity for gp120 expressed on the surface of infected cells. In this case, the uninfected cells bind, via the CD4 receptor-gp120 interaction, to infected cells and fuse to form a syncytium, which cannot survive. Destruction of CD4⁺ T-lymphocytes, which are critical to immune defense, is a major cause of the progressive immune dysfunction that is the hallmark of AIDS disease progression. The loss of CD4⁺ T cells seriously impairs the body's ability to fight most invaders, but it has a particularly severe impact on the defenses against viruses, fungi, parasites and certain bacteria, including mycobacteria.

Research on the Env glycoprotein has shown that the virus has many effective protective mechanisms with few vulnerabilities (Wyatt & Sodroski, Science. 1998 Jun 19;280(5371):1884-8). For fusion with its target cells, HIV-1 uses a trimeric Env complex containing gp120 and gp41 subunits (Burton et al., Nat Immunol. 2004 Mar;5(3):233-6). The fusion potential of the Env complex is triggered by engagement of the CD4 receptor and a coreceptor, usually CCR5 or CXCR4. Neutralizing antibodies seem to work either by binding to the mature trimer on the virion surface and preventing initial receptor engagement events, or by binding after virion attachment and inhibiting the fusion process (Parren & Burton, Adv Immunol. 2001;77:195-262). In the latter case, neutralizing antibodies may bind to epitopes whose exposure is enhanced or triggered by receptor binding. However, given the potential antiviral effects of neutralizing antibodies, it is not unexpected that HIV-1 has evolved multiple mechanisms to protect it from antibody binding (Johnson & Desrosiers, Annu Rev Med. 2002;53:499-518).

Data from most experimental HIV-1 vaccines tested in human and/or non-human primate suggest that a successful vaccine will incorporate immunogens that elicit broad neutralizing antibodies (bNAbs) and robust cell-mediated immunity. HIV-1 envelope glycoprotein (Env) is the main viral protein involved in the entry of the virus and is also the primary target for neutralizing antibodies, but due to immune evasion strategies and extreme sequence variability of Env, generation of bNAbs has been a daunting task (Phogat S, Wyatt R. Curr Pharm Des. 2007;13:213-27, Phogat S, et al. J Intern Med. 2007 262:26-43, Karlsson Hedestam GB, et al Nat Rev Microbiol. 2008 6:143-55).

The ability to elicit broad and potent neutralizing antibodies is a major challenge in the development of an HIV-1 vaccine. Namely, HIV-1 has evolved an impressive array of strategies to evade antibody-mediated neutralization, bNAbs develop over time only in a proportion of HIV-1 infected individuals, and only a handful of broad neutralizing monoclonal antibodies have been isolated from clade B infected donors to date. These isolated broad neutralizing monoclonal antibodies tend to display less breadth and potency against non-clade B viruses, and they recognize epitopes on the virus that so far have failed to elicit broad neutralizing responses when incorporated into a diverse range of immunogens. Presumably, this is due to the ability of these bNabs to recognize conserved recessed targets on HIV Env which are either inaccessible by the elicited antibodies or difficult to precisely redesign and present to the immune system.

Citation or identification of any document in this application is not an admission that such document is available as prior art to the present invention.

### SUMMARY OF THE INVENTION

As described above, the Env polypeptide is present on HIV-1 as a trimer; however, it is desirable at least in some instances to use monomeric polypeptides in the production of vaccines or immunogenic compositions. Therefore, it would be advantageous to identify Env polypeptides that are capable of binding to an antibody both as a trimer and as a monomer.

Applicants developed a panel of HIV-1 virus envelope genes for use in neutralization assays and epitope mapping projects. The panel was used to identify viral gp160 envelope monomers which bind to broadly neutralizing antibodies PGT145, PGT151 and PG9 MAbs and optionally PG16 and PGV04. The envelope sequence panel may be composed of single (clonal) envelope genes (such as, but not limited to SEQ ID NO: 1, 2, 3, 4 and/or 5) selected from the quasispecies present in HIV-infected plasma/sera.

A major goal in developing the envelope panel was to map the regions/residues of the envelope that are bound by neutralizing antibodies. This mapping could not be accomplished with the parental quasispecies vector preparations because the variation in the genetic sequences of the clones in the population made it impossible to generate clear nucleotide sequences. To perform the mapping, individual clones from the gene population were selected that yielded unambiguous sequence for the analyses.

In an advantageous embodiment, the soluble envelope glycoproteins of the present invention may be isolated from HIV-1 Clade A virus, HIV-1 Clade B virus, HIV-1 Clade C virus, a HIV-1 Clade A pseudo-virus, HIV-1 Clade B pseudo-virus or a HIV-1 Clade C pseudo-virus. All clades of Group M HIV (such as, but not limited to, Clade A, B, C, D, E (CRF01_AE), F, G(CRF02AG), H, I (CRF04_cpx), J, K) are contemplated as well as clades from other Groups of HIV.

In a particularly advantageous embodiment, or one or more components thereof of an env protein, may be prepared, purified and formulated for immunization in a human.

In another particularly advantageous embodiment, one or more components thereof of an env proteinmay be formulated for immunization in a human to contain an adjuvant.

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings.

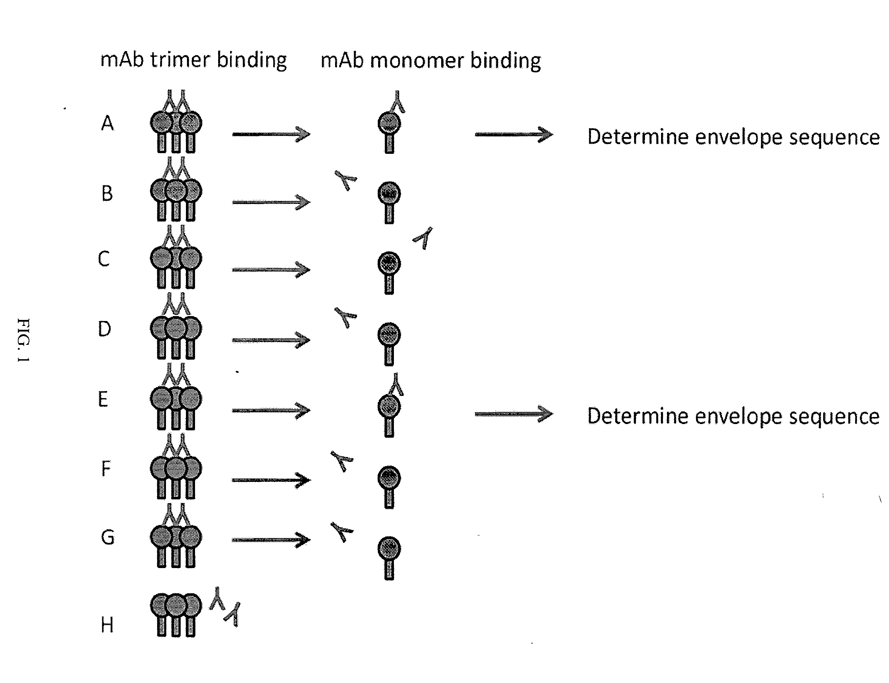

FIG. 1 depicts a schematic diagram illustrating one embodiment of certain methods disclosed herein. In these methods, monoclonal antibodies are first assayed to determine whether they can bind to several different envelope polypeptides present in trimeric form (shown in the left column of the schematic, with different envelope polypeptides A-H being tested). Because the monoclonal antibodies bound polypeptides A-G in trimeric form, these polypeptides were then assayed to determine whether the monoclonal antibodies bound the envelope polypeptides in monomeric form (center column of the schematic). The trimer form of the envelope polypeptides may be disrupted by any number of methods well known to one of ordinary skill in the art. (e.g., treatment with detergent). In this example, only monomers of envelope polypeptides A and G were bound by the monoclonal antibodies. Therefore, the nucleic acid sequences encoding these two envelope polypeptides is then determined.
FIG. 2 depicts binding of QNE specific mAbs to 293F expressed GNL-SEC purified recombinant gp120s.
FIG. 3 depicts binding of PGT140 series Abs to GNL MGRM2-gp120 (293S).
FIG. 4 depicts binding affinity of PG9, 16 antibodies to gp120s (SPR).

### DETAILED DESCRIPTION OF THE INVENTION

Without being bound by theory, Applicants hypothesize that within a collection of HIV isolates, there may exist a subset of isolates which may comprise building blocks and research tools for developing an HIV vaccine. Generally, this subset of isolates having such characteristics are believed to bind broadly neutralizing antibodies, such as but not limited to PG9, PG16, PGT145 and PGT151. The behavior of these sequences may be confirmed in binding assays to verify their characteristics as well as incorporating these sequences into constructs for research and immunogen design.

The sequence of a nucleic acid encoding an env may be

ATGAGAGTGATGGGGATACAGAGGAATTGTCCACTCTCATGGAGATGGGGTATGAT
GATATTTGGAATAATGATGATTTGTAGTGCTGCACAATTGTGGGTCACAGTCTACTA

TGGGATACCTGTGTGGAGAGACGCAGAGACCACCCTATTTGTGCATCAGATGCTAA
AGCCTATGATACAGAAGCTCATAATGTCTGGGCTACACATGCCTGTGTACCCACAGA
CCCTGACCCACAAGAAATACATTTGAAAAATGTAACAGAAAATTTTAACATGTGGA
AAAATGGCATGGTAGAGCAGATGCATGAAGATATCATTAGTCTATGGGACCAAAGC
CTAAAGCCATGTGTAAAGTTAACCCCTCTCTGCGTTACTTTAATTTGTAGCAATGTAA
CTAGTGGTAGCAATGTAACTAGTGGTAGCAATGTAACTAGTGGCAACAGCAACATA
TCTAATGAGATGGCTGGGGAAATAAAAAACTGCTCTTTCAATATGACCACAGAACT
AAGAGATAAGAAACAGAAAGTGTATGCACTTTTTATAGATCTGATGTGGAACCAA
TGGATAACAAGAGTGAGGAATATAGGTTAATATACTGTAATACCTCAACCATTGCAC
AGGCTTGTCCAAAGATAACCTTTGAGCCAATTCCAATACATTATTGTGCCCCAGCTG
GTTTTGCAATTCTAAAGTGTAATGATAAGGAATTCAATGGAATAGGGCCATGCAAG
AATGTTAGTACAGTACAATGCACACATGGAATCAAACCAGTAGTATCAACTCAACT
GCTACTAAATGGCAGTCTAGCAGAAGAAAAGATAGCAATCAGATCTGAAAATATCT
CAAACAATGCCAAAACCATAATAGTACAGTTGGCTACTCCTGTAAAAATTATTTGTA
CCAGACCTAACAACAATACAAGAAAGAGTATACGTATAGGACCAGGGCAAGCATTC
TATGCAGCAAATAAGATAATAGGGGATATAAGAAAAGCACACTGTAATGTCAGTAA
AGCAATATGGAATAACACTTTACAAAAGGTGGCTGAACAATTAAAGAAGCACTTTC
CGAATAAAACAATAGTCTTTGCTAACTCCTCAGGAGGGGATATAGAGATTACAACA
CATAGTTTTAATTGTGGAGGAGAATTTTTCTATTGCAATACATCAGACCTGTTTAATA
GCACTTGGGATAACAATACCAACAGTTCAAACTTCACAGGTAATGACACTATAACTC
TCCAATGCAGAATAAAGCAATTTGTAAATATGTGGCAGAGGGTAGGACAAGCAATG
TATGCCCCTCCCATCGAAGGAAGAATAAGATGTGAATCAAATATTACTGGACTACTA
TTAACAAGAGATGGAGGAGAAGGTAATAATAGGACAAATGAAACCTTCAGGCCTGG
AGGAGGAGATATGAGGGACAATTGGAGAAGTGAATTATATAAGTATAAAGTAGTAA
AAATTGAACCACTAGGTGTAGCACCCACCCATGCAAAAAGAAGAGTGGTGCAGAGA
GAAAAAAGAGCAGTGGGACTGGGAGCTGTCTTCCTTGGGTTCTTAGGAGCGGCAGG
AAGCACTATGGGCGCGGCGTCAATAACGCTGACGGTACAGGCCAGACAATTATTGT
CTGGTATAGTGCAACAGCAGAGCAATTTGCTGAAGGCTATAGAGGCTCAACAACAT
CTGTTGAAACTCACAGTCTGGGGCATTAAACAGCTCCAGGCAAGAGTCCTGGCTCTA
GAAAGGTACCTAAAGGATCAACAGCTCCTAGGAATATGGGGCTGCTCTGGAAAACT
CATCTGTACCACCACTGTACCCTGGAACTCTAGTTGGAGTAATAAAACCTATGAGGA

CATATGGGATAACATGACCTGGATACAATGGACAGAGAAATTAGCAATTACACAA
ACAAAATATATGAGCTACTTGAAGAATCGCAGAACCAGCAGGAAAAGAATGAACA
AGACTTATTGGCATTAGACAAGTGGGCAAGTCTGTGGAATTGGTTTAACATATCAAA
TTGGTTATGGTATATAAAAATATTTATAATGATAGTAGGAGGCTTGATAGGTTTAAG
AATAATTTTTGCCGTGCTTACTATAATAAATAGAGTTAGGCAGGGATACTCACCTCT
GTCGTTCCAGACCCTTACCCACCACCAGAGGGAACCCGACAGGCCCAGAAGAATCG
AAGAAGGAGGTGGCGAGCAAGACAGAGACAGATCCGTGCGATTAGTGAGCGGATT
CTTAGCGCTTGCTTGGGACGATCTGCGGAGCCTGTGCCTCTTCAGCTACCACCGATT
GAGAGACTTTGTCTTGATTCTGGGACACAGCGGTCTCAAGGGACTGAGACTGGGGT
GGGAAGCCCTCAAATATCTGTGGAATCTTCTATCATACTGGAGTCAGGAACTAAAGA
ATAGTGCTATTAGCTTGCTTAATACAATAGCAATAGCAGTAGCTAATTGGACAGACA
GAGTTATAGAAATAGTACAAAGAGCTGGTAGAGCTATTTGCAACATACCTAGAAGA
ATTAGACAGGGGCTTGAGAGATCTTTGCTATAA (SEQ ID NO: 1);

ATGAGAGTGATGGGGATACAGAGGAATTGTCCACTCTCATGGAGATGGGGTATGAT
GATATTTGGAATAATGATAATTTGTAGTGCTGCACAATTGTGGGTCACAGTCTACTA
TGGGGTACCTGTGTGGAGAGACGCAGAGACCACCCTATTTTGTGCATCAGATGCTAA
AGCCTATGATACAGAAGCTCATAATGTCTGGGCTACACATGCCTGTGTACCCACAGA
CCCTGACCCACAAGAAATACATTTGAAAAATGTAACAGAAGATTTTAACATGTGGA
AAAATGGCATGGTAGAGCAGATGCATGAAGATATCATTAGTCTATGGACCAAAGC
CTAAAGCCATGTGTAAAGTTAACCCCTCTCTGCGTTACTTTAAATTGTAGCAGCAAT
GTAACTAGTGGCAACAGCAGCATACCTGAGGAGATGTCTGGGGTAAAAAACTGCTC
TTTCAATATGACCACAGAACTAAGAGATAAGAAACAGAAAGTGTATGCACTTTTTTA
TAGATCTGATGTGGAACTAATGGATAACAACACGAGTGAATATAGGTTAATAAATT
GTAATACCTCAGCCATTGCACAGGCTTGTCCAAAGATAACCTTTGAGCCAATTCCAA
TACATTATTGTGCCCCAGCTGGTTTTGCAATTCTAAAGTGTAATGATGAGAACTTCA
ATGGAACAGGGCCATGCAAGAATGTTAGTACAGTACAATGCACACATGGAATCAAA
CCAGTAGTATCAACTCAACTGCTACTAAATGGCAGTCTAGCAGAAGGAAAGATAGC
AATCAGATCTGAAAATATCTCAAACAATGCCAAAACCATAATAGTACAGTTGGTTAC
TCCTGTAAAAATTACTTGTACCAGACCTAACAACAATACAAGAAAGAGTATACGTAT
AGGACCAGGGCAAGCATTCTATGCAGCAAATAAGATAATAGGGGATATAAGAAAA
GCACACTGTAATGTCAGTAAAGCACTATGGAATAACACTTTACAAAGGTGGCTGA

ACAATTAAAGAAGCACTTTCCGAATAAAACAATAGTCTTTGCTAACTCCTCAGGAGG
GGATATAGAGATTACAACACATAGTTTAATTGTGGAGGAGAATTTTTCTATTGCAA
TACATCAGACCTGTTTAATAGCACTTGGGATAACAATACCAACAGTTCAAACTTCAC
AGGTAATGACACTATAACTCTCCAATGCAGAATAAAGCAATTTGTAAATATGTGGCA
GAGGGTAGGACAAGCAATGTATGCCCCTCCCATCGAAGGAAAAATAAAATGTCAAT
CAAATATTACTGGACTACTATTAACAAGAGATGGAGGAGAAGGTAATAATAGGACA
AATGAAACCTTCAGGCCTGGAGGAGGAGATATGAGGGACAATTGGAGAAGTGAATT
ATATAAGTATAAAGTAGTAAAAATTGAACCACTAGGAGTAGCACCCACCCATGCAA
AAAGAAGAGTGGTGACGAGAGAAAAAAGAGCAGTGGGACTGGGAGCTGTCTTCCTT
GGGTTCTTAGGAGCAGCAGGAAGCACTATGGGCGCGGCGTCAATAACGCTGACGGT
ACAGGCCAGACAATTATTGTCTGGTATAGTGCAACAGCAGAGCAATTTGCTGAAGG
CTATAGAGGCTCAACAACATCTGTTGAAACTCACAGTCTGGGGCATTAAACAGCTCC
AGGCAAGAGTCCTGGCTCTAGAAAGGTACCTAAAGGATCAACAGCTCCTAGGAATA
TGGGGCTGCTCTGGAAAACTCATCTGTACCACCACTGTACCCTGGAACTCTAGTTGG
AGTAATAAAACCTATGAGGACATATGGGATAACATGACCTGGATACAATGGGACAG
AGAAATTAGCAATTACACAAATAAAATATATGAGCTACTTGAAGAATCGCAGAACC
AGCAGGAAAAGAATGAACAAGACTTATTGGCATTAGACAAGTGGGCAAGTCTGTGG
AATTGGTTTAACATATCAAATTGGTTATGGTATATAAAAATATTTATAATGATAGTA
GGAGGCTTGATAGGTTTAAGAATAATTTTTGCTGTGCTTACTGTAATAAATAGAGTT
AGGCAGGGATACTCACCTCTGTCGTTCCAGATCCTTACCCACCACCAGAGGGAACCC
GACAGGCCCAGAAGAATCGAAGAAGGAGGTGGCGAGCAAGACAGAGACAGATCCG
TGCGATTAGTGAGCGGATTCTTAGCGCTTGCTTGGGACGATCTGCGGAGCCTGTGCC
TCTTCAGCTACCACCGATTGAGAGACTTTGTCTTGATTCTGGGACACAGCAGTCTCA
AGGGACTGAGACTGGGGTGGGAAGCCCTCAAATATCTGTGGAATCTTCTATCATACT
GGAGTCAGGAACTAAAGAATAGTGCTATTAGCTTGCTTAATACAACAGCAATAGCA
GTAGCTAATTGGACAGACAGAGTTATAGAAATAGGACAAAGAGCTGGTAGAGCTAT
TCGCAACATGCCTAGAAGAATTAGACAGGGCCTTGAGAGATCTTTACTATAA (SEQ
ID NO: 2);

ATGAGAGTGAGGGGGATGCAGAGGAATTATCAGCACTTGGTGAAGTGGGGCCTCTT
GTTCTTGGGAATATTAATAATCTGTAATGCTACTGATAACTTATGGGTCACAGTATA
TTATGGGGTACCTGTGTGGAGAGAAGTATCCACTACTCTATTCTGTGCATCAGATGC

CAAAGCATATGACAAGGAGGTACATAATGTCTGGGCTACACATGCCTGTGTACCCA
CAGACCCCAATCCACAAGAGGTAGTTCTGAAAAATGTAACAGAAAATTTTAATATG
TGGGAAAATAACATGGTAGAACAAATGCATACAGATATAATTAGTTTATGGATCA
AAGCCTAACCCCATGTGTGAAGTTAACCCCACTCTGTGTCACATTAAATTGTAGTGA
TGCCAAAAACAACACAGAGGTAAAACAACATGACACCCTGAAGGAAGAGGCAGGG
GCAATAAAAAACTGTTCTTTCAATATGACCACAGAAGTAAGAGATAAGCAGCTGAA
AGTATATGCACTCTTTTATAGGCTTGATATAGTACCAATCAGCAATAGCGATAGCAG
TAGTAAATATAGGCTAATAAATTGTAATACTTCAACCATTACACAGGCTTGTCCAAA
GGTATCTTGGGATCCAATTCCCATACATTATTGTGCTCCAGCTGGTTATGCGATTCTA
AAGTGTAATGAAAAAGACTTCAATGGAACAGGGCCATGCAAGAATGTCAGCACAGT
ACAATGTACACATGGAATTAAACCAGTGGTATCAACTCAATTGTTGTTAAATGGCAG
CCTATCAGAGGGAGATATAATAATCAGATCTCAAAATATCTCAGATAATGCAAAAA
CCATAATAGTTCACTTTAATGAATCTGTGCAGATTAATTGTACAAGACCCAACAACA
ATACAAGAAAAGGTATACATTTAGGACCAGGAAAAACATTCTATGCAACAGGGGAC
ATAATAGGAGACATCAGAAAGGCACATTGTAACATTAGTGGAGAACACTGGAATGA
GACTTTAGGAAAAGTAAAGACAAAGTTAGGGATTCTTTTCCCTAATAAAACAATAA
CATTTAATTCATCTTCAGGAGGAGATCTAGAAGTTACGATGCATAGTTTTAATTGTA
GAGGAGAATTTTTCTACTGCAATACATCAGGTCTGTTTAATAACACACTAAGCAATG
GCACCATCATTCTTCCGTGTAGAATAAAACAGATTGTAAACATGTGGCAGGAAGTA
GGACGAGCAATGTATGCCGCTCCCATTGCAGGAGAAATTATCTGTAGATCAAATATT
ACAGGTCTACTATTGACAAGAGATGGTGGTCAAAACATAACTGATCAAAATAAAAC
TGAGATCTTCAGACCTGGGGGAGGAAATATGAAAGACAATTGGAGAAGTGAACTAT
ATAAATATAAAGTAGTAGAAATTGAACCACTAGGTGTAGCACCCACCAGGGCAAAA
AGACAAGTGGTGAGCAGAGAAAAAAGAGCAGTGGGAACCCTGGGAGCTTTGTTCCT
TGGATTCTTGGGAACAGCAGGAAGCACTATGGGCGCGGCGTCAATAACGCTGACGG
TACAGGCCAGACAATTATTGTCTGGAATAGTGCAACAGCAGAACAATCTGCTGAGG
GCTATTGAAGCGCAACAGCATCTGTTGCAGCTCACAGTCTGGGGCATTAAACAGCTC
CAGGCAAGAGTCCTGGCTGTAGAAAGATACCTAAAGGATCAACAGCTCCTAGGGCT
TTGGGGCTGCTCTGGAAAGCTCATCTGCACCACTAATGTACCCTGGAATAATAGTTG
GAGTAATAAATCTCAGGAGGACATTTGGAACAACATGACCTGGATGCAATGGGACA
AGGAGATTAGTAATTACTCACAAGAAATATACAGGTTAATTGAAATATCGCAAAAC

CAGCAGGAAATAAATGAAAAGGAATTATTGGAGTTGGACAAGTGGGCAAGTCTGTG
GAATTGGTTTGACATATCAAATTGGCTGTGGTATATAAAAATATTCATAATGATAGT
AGGAGGCTTGATAGGCTTAAGAATAGTTTTTACTGTGCTTTCTGTAGTAAATAGAGT
TAGGAAGGGATACTCACCTTTGTCATTGCAGACCCTCCTCCCAAGCCCGAGGGGACC
CGACAGGCCCGAAGGAACAGAAGAAGGAGGTGGAGAGCAAGACAAAAACAGATCC
ATCAGATTAGTGAACGGATTCTTAGCTCTTGCCTGGGACGACCTGAGGAACCTGTGC
CTCTTCTGCTACCGCCAATTGAGAGACTTGATATTAATTGCAGCGAGAGTTGTGAAC
AGGGAACTGAGGGGGGTGTGGGAAGTCCTCAAGTATTTGGGGAATCTCACGCAGTA
CTGGATTCAGGAACTAAAGAATAGTGCTATTAGCTTGTTTAATACCACAGCAATAGT
AGTAGCTGAGGGAACAGATAGAATTATAGAGATTTTGCAAAGAGCTGGTAGAGCTA
TTCTCAACATACCTAGAAGAATAAGACAGGGCGCAGGAAGAGCTTTGCTATAA (SEQ
ID NO: 3);

ATGAGAGTGATGGAGATCAGGAAGAATTATCAGCAATGGTGGAAAGGGGGCATCTT
GCTCCTTGGGATGTTAATGATCTGTAGTACTGCAGAAAATTTGTGGGTCACAGTCTA
TTATGGAGTACCAGTGTGGAAAGAAACAACCACCACCTTATTTTGTGCATCAGATGC
TAAAGGATATGATACAGAGGCACATAATGTTTGGGCCACACATGCCTGTGTACCCAC
AGACCCCAGCCCACAAGAAGTAGTATTGGAAAATGTGACAGAAAATTTTAACATGT
GGAAAAATAACATGGTAGAACAAATGCATGAGGACATAATTAGTTTATGGGATCAA
AGCCTAAAGCCATGCGTAAAACTAACCCCACTCTGTGTTACTTTAAATTGCACTGAT
GAGGTTGGGAATACTACTAATACCACTAGTGGTAGCTGGGAGAAAACAATAGAAAA
GGGAGAAATAAAAAACTGCTCTTTCAATATCACCACAAACATAAGAGGTAAGATAC
AGAAACAATATGCACTTTTTCTGAACTTGATGTAGTACCAATGGATAATGATACTA
ACTATAGGTTGATAAGTTGTAACACTTCAGTCATTACACAGGCCTGTCCAAAGGTAT
CCTTTGAGCCAATTCCCATACATTTTTGTGCCCCGGCTGGTTTTGCGATTCTAAAGTG
TAACAATAAGACGTTCAATGGAAAAGGACCGTGTACAAATGTCAGCACAGTACAAT
GCACACATGGAATTAAGCCAGTAGTATCAACTCAACTGCTGTTAAATGGCAGCCTAG
CAGAAGAGGTAATTATTAGATCTGACAATTTCACGGACAATGCTAAAACCATAATA
GTACAGCTGAAGGAACCTGTAGAAATTAACTGTACAAGACCCAACAACAATACAAG
AAAAGGTATACATATAGGACCAGGGAGAGCCTTTTATGCAACAGGAGATATAATAG
GAGATATAAGAAAAGCATATTGTAATATTAGTCTTACAAAATGGAATAACACTTTAG
GACAGATAGTGAAAAAATTAAGAGAACAATTTGGGAATAAAACAATAATTTTTAAT

CAATCCTCAGGAGGGGACCCAGAAATTACAATGCACACCTTTAATTGTGGAGGGGA
GTTTTTCTACTGTAATTCAACAATACTGTTTAATAGTACTTGGCTGTCTAATAGTACT
TGGAATGAAACTATTACTGAAGGGGTAAATGTAAATGACACTATTATGCTCCCATGC
AGAATAAAGCAAGTCATAAACATGTGGCAGGAAGTAGGAAAAGCAATGTATGCCCC
TCCCATAAGAGGACGAATTAGATGTTCATCAAATATTACAGGGCTGATATTAACAAG
AGATGGTGGTAATAATCAGAAGAACAACGCCACAGAGACCTTCAGACCTGGAGGAG
GAGATATGAGGGACAATTGGAGAAGTGAATTATATAAATATAAAGTAGTACAAATT
GAACCAGTAGGAGTAGCACCCACCAAGGCAAAGAGAAGAGTGGTGCAAAGAGAAA
AAAGAGCAGTGGGAATAGGAGCTATGTTCATTGGGTTCTTGGGAGCAGCAGGAAGC
ACTATGGGCGCAGCGTCAATGACGCTGACGGTACAGGCCAGACAATTGTTGTCTGGT
ATAGTGCAACAGCGGAACAATCTGCTGAGGGCTATTGAGGCGCAACAGCACCTGTT
GCAACTCACAGTCTGGGGCATTAAGCAGCTCCAGGCAAGACTCCTGGCTGTGGAAA
GATACCTAAAAGATCAACAGCTCCTGGGGTTGTGGGGTTGCTCTGGAAAACTCATTT
GCACCACTACTGTACCTTGGAATGCTAGTTGGAGTAATAAATCTCTGAACACTATTT
GGAATAACATGACCTGGATGCAGTGGATAGAGAAATTGACAACTACACAAACCTA
ATATACAACTTAATTGCAGAATCGCAGAACCAGCAAGAAAAGAATGAACAAGAACT
ATTGGAATTAGATAAATGGGCAAGTTTGTGGAATTGGTTTAGCATAACAAATTGGCT
GTGGTATATAAAAATATTCATAATGATAGTAGGAGGCCTAGTAGGTTTAAGAATAGT
CCTTACTGTACTTTCTATAGTGAATAGAGTTAGGCAGGGATACTCACCATTATCGTTT
CAGACCCGCCTCCCAACCCAGAGGGGACCCGACAGGCCCGGAGGAATCGAAGAAG
AAGGTGGCGAGAGAGACAGAGACAGATCCGGTCCCTCAGCGGATGGCTTCTTAGCA
ATTATCTGGGTCGATCTGCGGAGCCTGTGCCTCTTCAGTTACCACCACTTGAGAGAC
TTACTCTTGATTGTAACGAGGATTCTGGAACTTCTGGGACGCAGGGGGTGGAAGCC
CTCAAATATTGGTGGAACCTGATACAGTATTGGAGTCAGGAACTAAAGAATAGTGC
TGTTAGCTTGTTCAACGCCATAGCCATAGCAGTAGCTGAGGGAACAGATAGGATTAT
AGAAATATTACAAAGAGGTTTTAGAGCTGTCCTCCACATACCTAGAAGAATAAGAC
AGGGCTTGGAAAGGGCTTTGCTATAA (SEQ ID NO: 4); and/or

ATGAGAGTGATGGGGATACAGAAGAATTATCCACTCTTTTGGAGATGGGGTGTGAT
AATATTTTGGATAATAATAATTTGTAATGCTAATCAGTTGTGGGTCACGGTCTACTAT
GGGGTACCTGTGTGGAGAGATGCAGATACCACCCTATTTTGTGCATCAGATGCTAAA
GCATATGATAAAGAAGTACACAATGTCTGGGCCACACATGCCTGTGTACCTACAGA

CCCCAACCCACAAGAAATACATTTGGAAAATGTAACAGAAAATTTTAACATGTGGA
AAAATAACATGGTAGAACAGATGCATGAAGATATAATTAGTCTATGGGACCAAAGC
CTACAGCCATGTGTAAAGTTAACCCCTCTCTGTGTTACTTTAAATTGCAGTAATAGCT
ATAACACCAGTAAAGTTAACATTACTGAGGGGATGAACGAGGAAATAAAAAACTGT
TCTTTCAATATGACCACAGAATTAAGAGATAAGAGAAGGAAAGAGTATGCACTTTTT
TATAAACTTGATATAGTACAAATTAATGAAGGAAAAAATAACAGTAATAATAATAA
TACTCAGTATATGTTAATAAATTGTAATACCTCAGCTATTACACAGGCTTGTCCAAA
GGTGACCTTTGAGCCCATTCCCATACATTATTGTGCCCCAGCTGGTTTTGCGATTCTA
AAGTGTAATGAGAAGAAGTTCAATGGAACAGGGCCATGCCAGAATGTCAGTACAGT
ACAATGCACACATGGAATCAAGCCAGTAGTATCAACTCAACTGCTATTAAATGGCA
GTCTAGCAGAAGAAGAAATAGTGATTAGATCTGAAAATATCACAAATAATGCCAAA
ACCATAATAGTACAGCTGGCTGCGCCTGTAAAAATTAATTGTATCAGACCAGGCAAC
AATACAAGAAGAAGTGTACGTATAGGACCAGGGCAAACCTTCTTTGAAACAGGTGA
CATAATAGGGGATATAAGACAAGCACATTGTAATGTCAATAGAACAGCTTGGAATG
ACACTTTAAGACAGGTAGCTAGACACTTAGGGGGGTACTTTAATAATAATACAATA
AAGTTTACTAACCACTCAGGAGGGGATTTAGAAATTACAACACATAGTTTTAATTGT
GGAGGAGAATTTTTCTATTGCAATACATCAAATCTGTTTAATAGCACTTGGAACAAT
AGCACTTGGAATAACAGTAACAATGCCAGTACAAACCAAACAGATGACATTATAAT
ACTCCAATGCAGGATAAAGCAGATTGTAAATATGTGGCAGAGAGTAGGACAAGCAA
TGTATGCCCCTCCCATCCAAGGAAACATAAGCTGTAAATCAAACATTACTGGATTAC
TATTAACAAGAGATGGAGGGAGTAGCAGTAATGGAACTACTGAGACCTTCAGGCCT
GGAGGAGGAGACATGAGAGACAATTGGAGAAGTGAATTATATAAGTATAAAGTAGT
AAAAGTTGAACCCCTAGGTGTAGCACCCACCCATGCAAGAAGAAGAGTGGTGAAGA
GAGAAAAAAGAGCAGTTGGACTGGGAGCTGTCTTCTTTGGGTTCTTAGGAGCAGCA
GGAAGCACTATGGGCGCGGCGTCAATAACGCTGACGGTACAGGCCAGACAATTACT
GTCTGGTATAGTGCAACAGCAGAGCAATCTGCTGAAGGCTATAGAGGCTCAACAGC
ATCTGTTGAGACTCACGGTCTGGGGCATTAAACAGCTCCAGGCAAGAGTCCTGGCTC
TGGAAAGTTACCTAAAAGATCAACAGCTTCTAGGAATTTGGGGCTGCTCTGGAAAA
CTCATCTGCACCACTACTGTACCCTGGAACTCTAGTTGGAGTAGTAGAACTTATGAG
AGCATATGGAATAACATGACCTGGCTGCAATGGGATAAAGAAGTTAGCAATTACAC
AGACATAATATATGCTCTAATTGAAGAATCGCAGAACCAGCAGGAAAAAATGAAC

AAGACTTATTGGCATTGGACAAGTGGGCAAGCCTGTGGACTTGGTTTGGCATAACAA
ACTGGCTGTGGTATATAAAAATATTTATAATGATAGTAGGAGGTTTAATAGGTTTAA
GAATAGTTTTTGCTGTGCTTTCTATAATAAATAGAGTTAGGCAGGGCTACTCACCTTT
ATCATTTCAGATCCTTACCCACCACCAGAGGGAACCCGACAGGCCCGGAAGAACCG
AAGAAGAAGGTGGCGAGCAAGACAGAGACAGATCCGTGAGATTAGTGAGCGGATT
CTTAGCACTTGCCTGGGACGACCTGCGGAACCTGTGCCTCTTCTGCTACCACCGATT
GAGAGACTTTCTCTTGATTGTAGCGAGGACTGTGGAACTCCTGGGACACAGCAGTCT
CAAGGGGCTGAGGCAGGGGTGGGAAGCCCTCAAAATTCTGGGGAATCTTCTATCAT
ACTGGGGCCAGGAACTAAAGAATAGTGCTATTAATTTACTTGATACAATAGCAATA
GCAGTAGCTAACTGGACAGACCGAGTAATAGAAATAGGACAAAGAGTTGGTAGAG
GCATTCTCAACATACCTAGAAGAATCAGACAGGGCCTCGAAAGGGCTTACTATAAA
AT (SEQ ID NO: 5).

In a particularly advantageous embodiment, the nucleic acid sequences of the envelope glycoproteins of the present invention may have about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to any one of SEQ ID NOS: 1-5.

The present invention also encompasses proteins encoded by the nucleic acids of any one of SEQ ID NOS: 1-5.

In a particularly advantageous embodiment, the soluble envelope glycoproteins of the present invention have about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to a polypeptide encoded by any of the sequences depicted in the specification, such as SEQ ID NOS: 1-5.

Assays for screening for neutralizing antibodies are known in the art. One neutralization assay approach has been described previously (Binley JM, et al., (2004). Comprehensive Cross-Clade Neutralization Analysis of a Panel of Anti-Human Immunodeficiency Virus Type 1 Monoclonal Antibodies. J. Virol. 78: 13232-13252). Pseudoviruses may be generated by co-transfecting a first cell with at least two plasmids, one plasmid having the nucleic acid sequence encoding the Env polypeptide of the present invention and the other plasmid including the rest of the HIV genome. In the HIV genome encoding vector, the env sequence may be replaced by the firefly luciferase gene. The transfected cells are incubated, and then pseudoviruses comprising the Env polypeptide of the present invention enter the supernatant of the cells. The supernatants containing pseudoviruses can be used in a neutralization assay. The supernatants may be co-incubated for 1 hour or overnight with a neutralizing antibody preparation or B cell supernatants comprising antibodies derived from activation of an infected donor's primary peripheral blood mononuclear cells (PBMCs). Test cells (cells stably transfected with and expressing CD4 plus the CCR5 and CXCR4 coreceptors) may be added to the mixture and incubated for 3 days at 37° C, allowing any pseudoviruses that have not been neutralized by the antibodies, to infect the test cells. Infected test cells may be quantified by luminometry.

In another embodiment of the present invention, the neutralizing antibodies may be PGT145, PGT151, PG16, PG9 or PGV04 or any other neutralizing antibodies disclosed in international patent publication WO 2012/030904).

Applicants developed a panel of HIV-1 virus envelope genes for use in neutralization assays and epitope mapping projects. The panel was used to identify viral gp160 envelope monomers which bind to the PGT145, PGT151 and PG9 MAbs. The envelope panel may be composed of single (clonal) envelope genes selected from a quasispecies present in HIV-infected plasma/sera. Development and characterization of the envelope panel (quasispecies) may include RT-PCR of viral env genes present in a clinical sample (plasma), cloning of the genes into an expression vector, expansion of the vector in bacterial culture and purification of the vector DNA, generation of pseutodype virus stocks and characterization of the pseudotype stocks: infectivity, cell co-receptor usage (CCR5 and/or CXCR4) and sensitivity to neutralization by a panel of monoclonal antibodies (MAb) and polyclonal HIV+ plasma/sera. An illustrative panel is presented in Table 1.

Table 1: Multiclade gp160 Clone Panel: Binding ELISA with Mab Titration. The numbers in the table indicate the ELISA OD or IC50 (where indicated) The heat code map indicates a <cutoff = OD <0.2, Median OD = 0.2-0.25, Median OD = 0.25-0.35 and Median OD = >0.35.

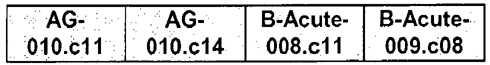

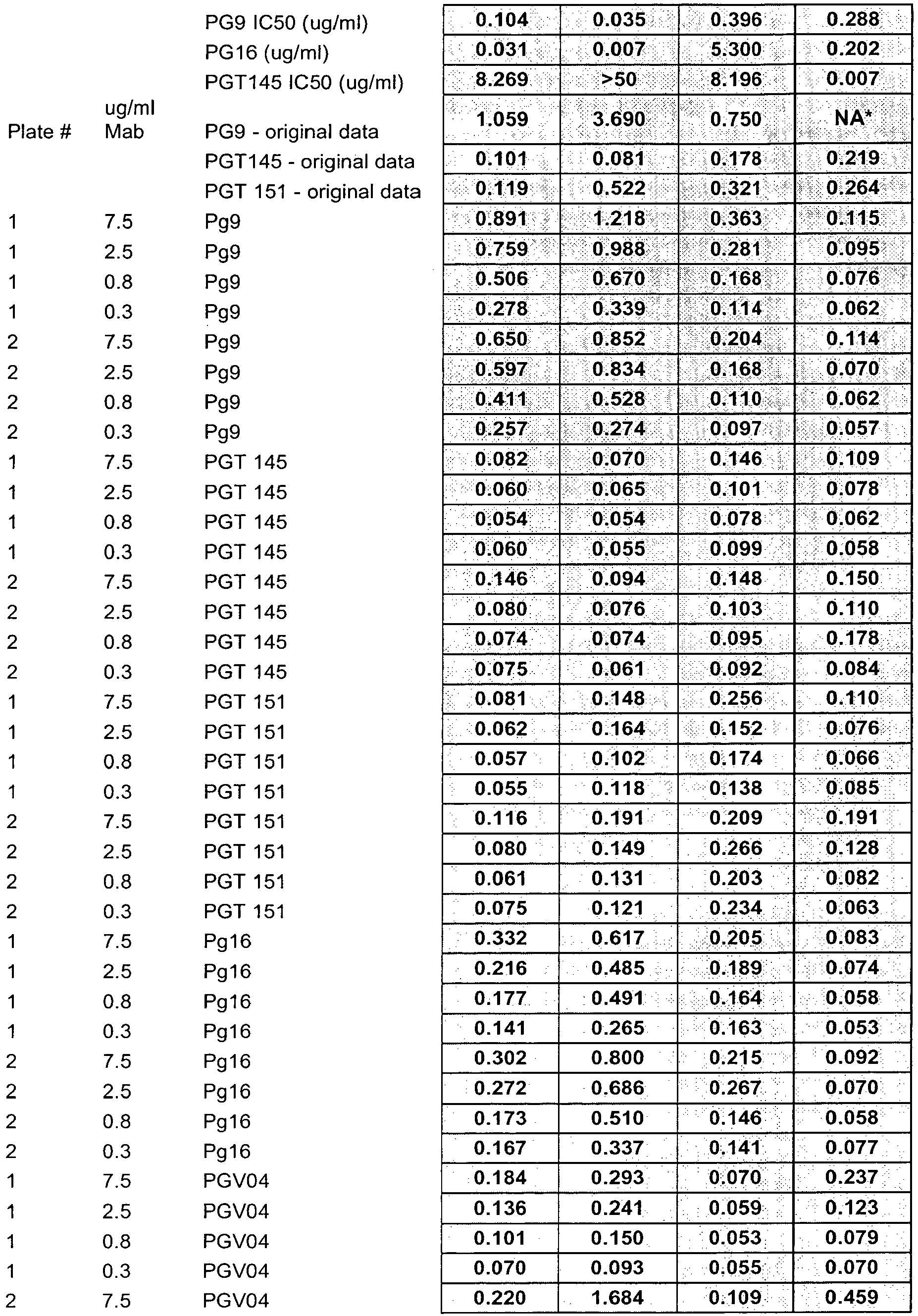

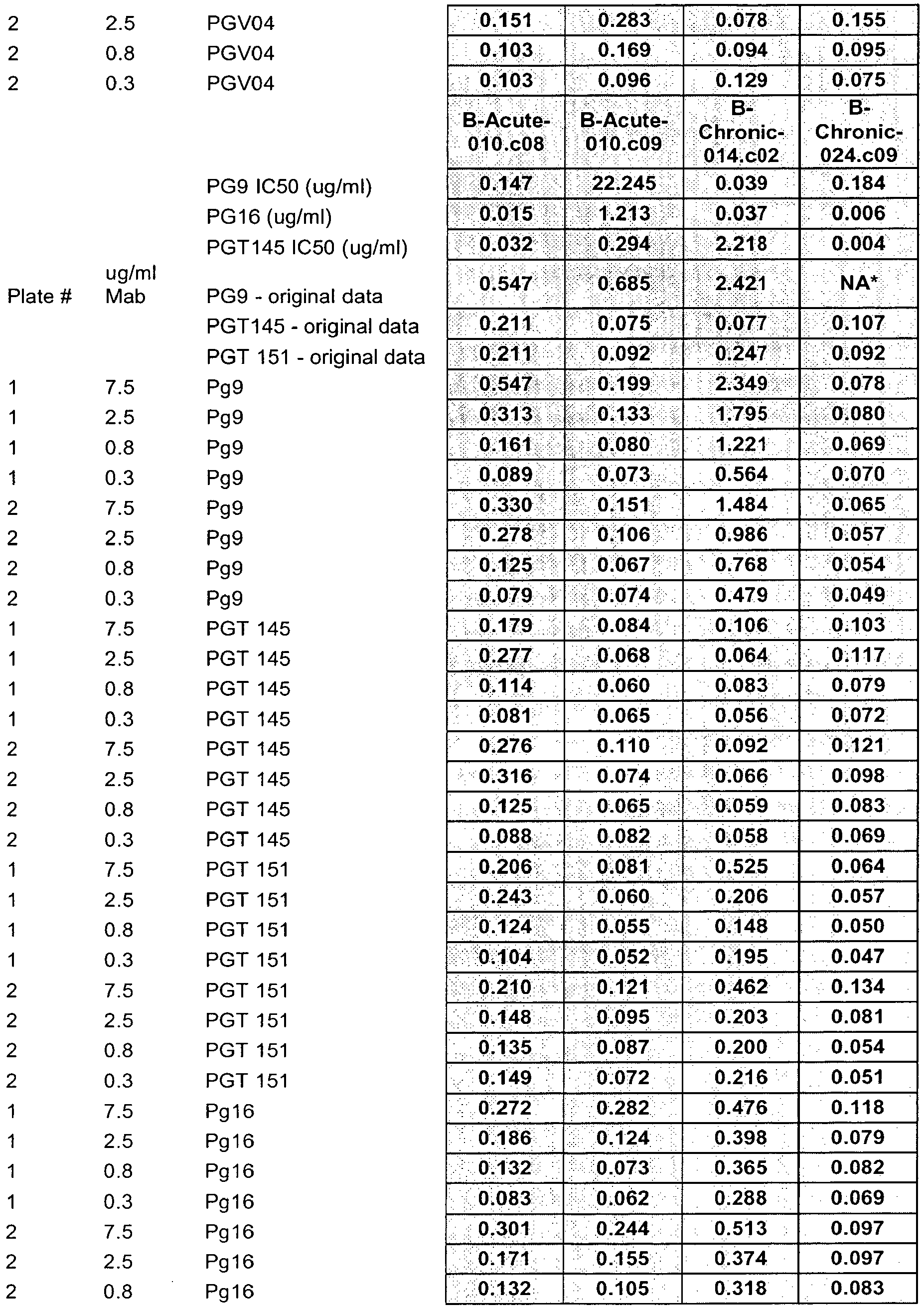

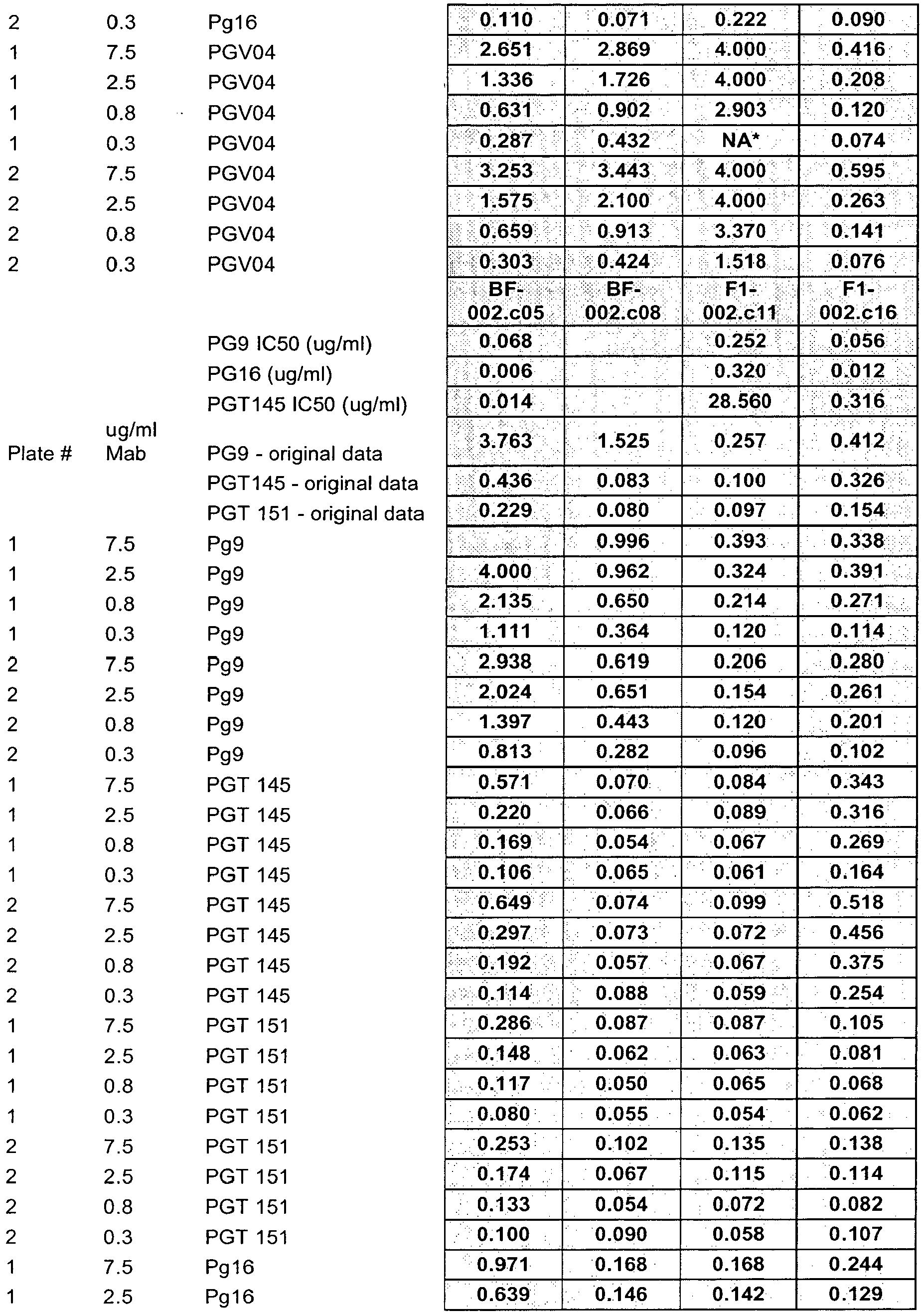

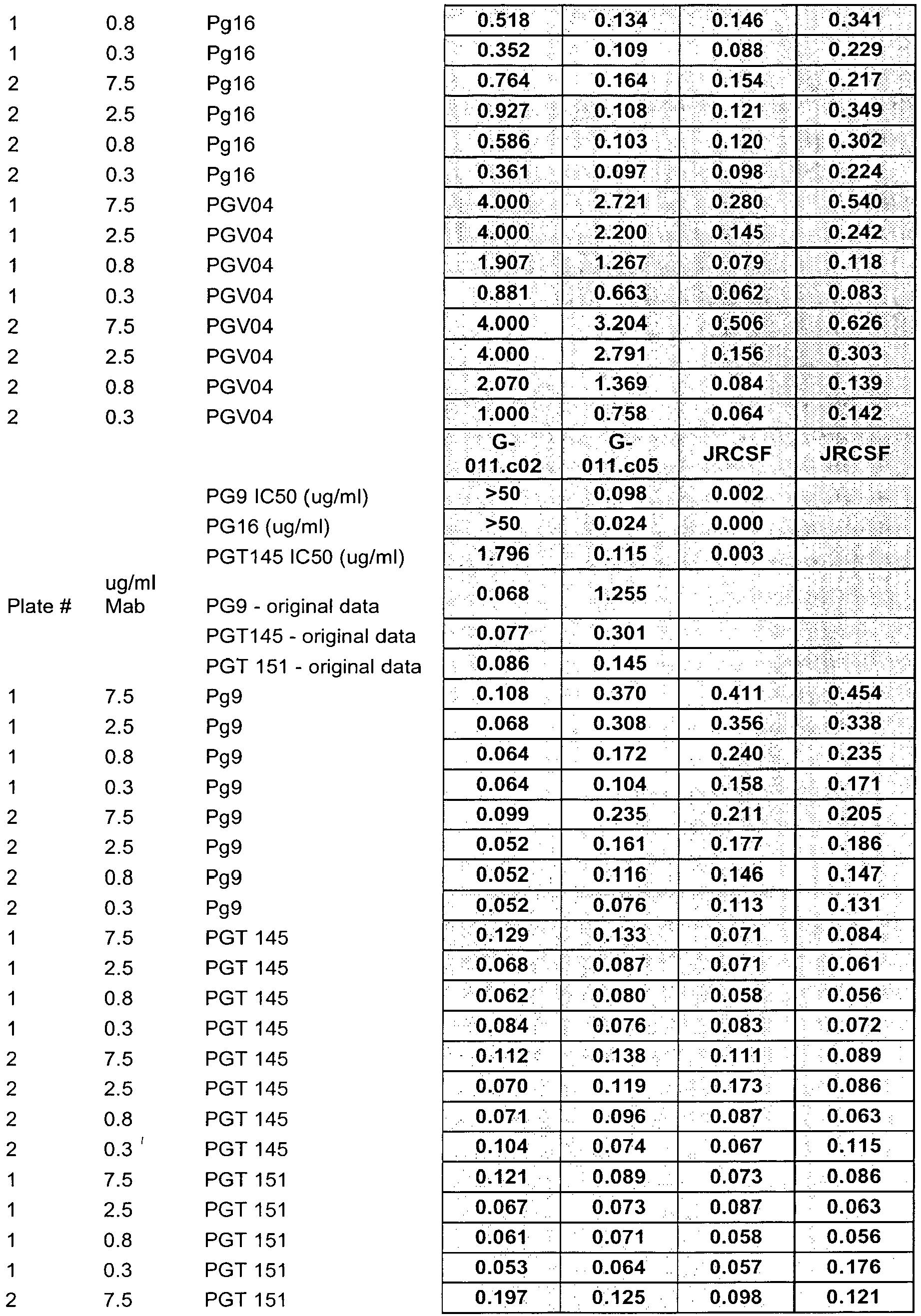

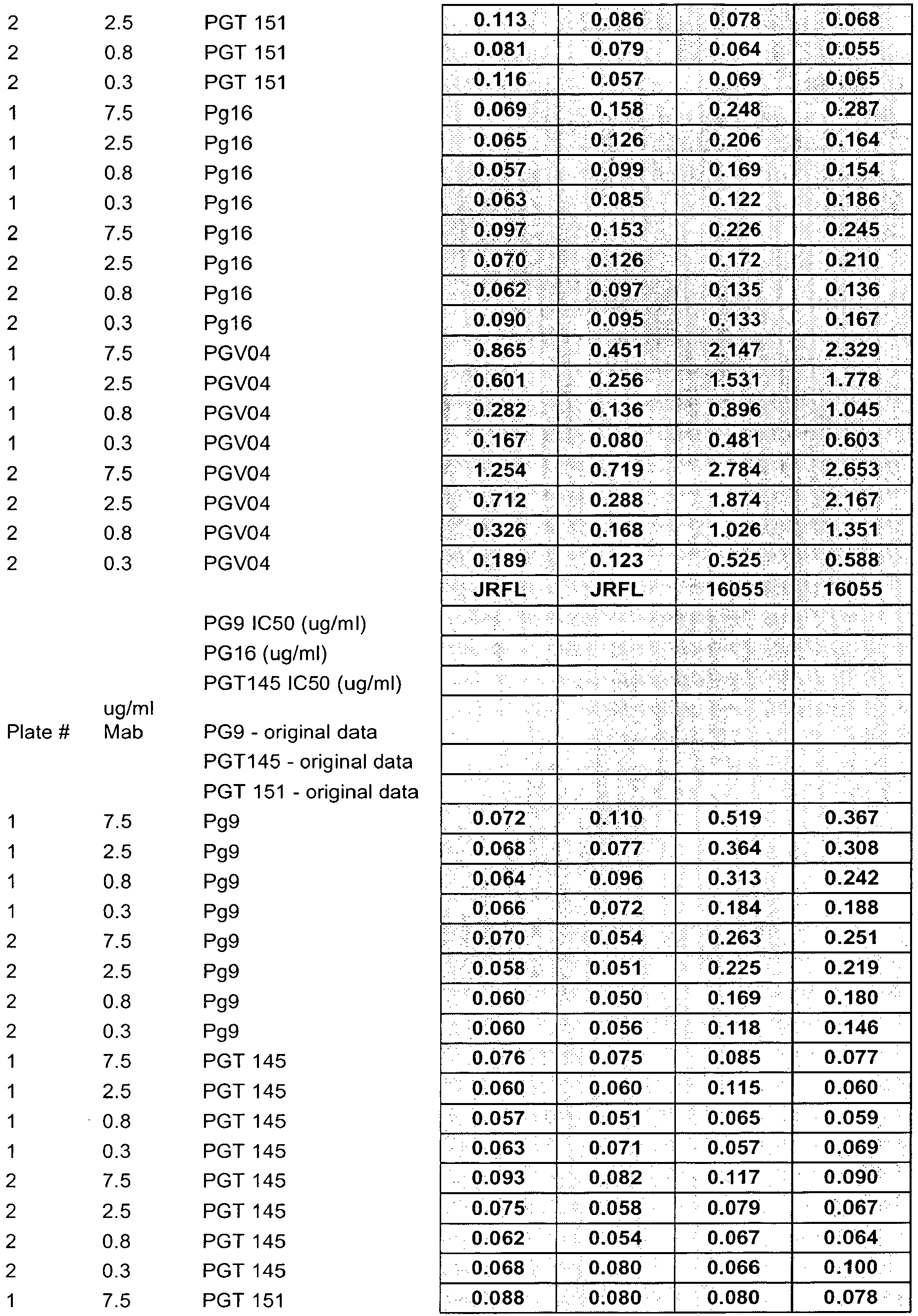

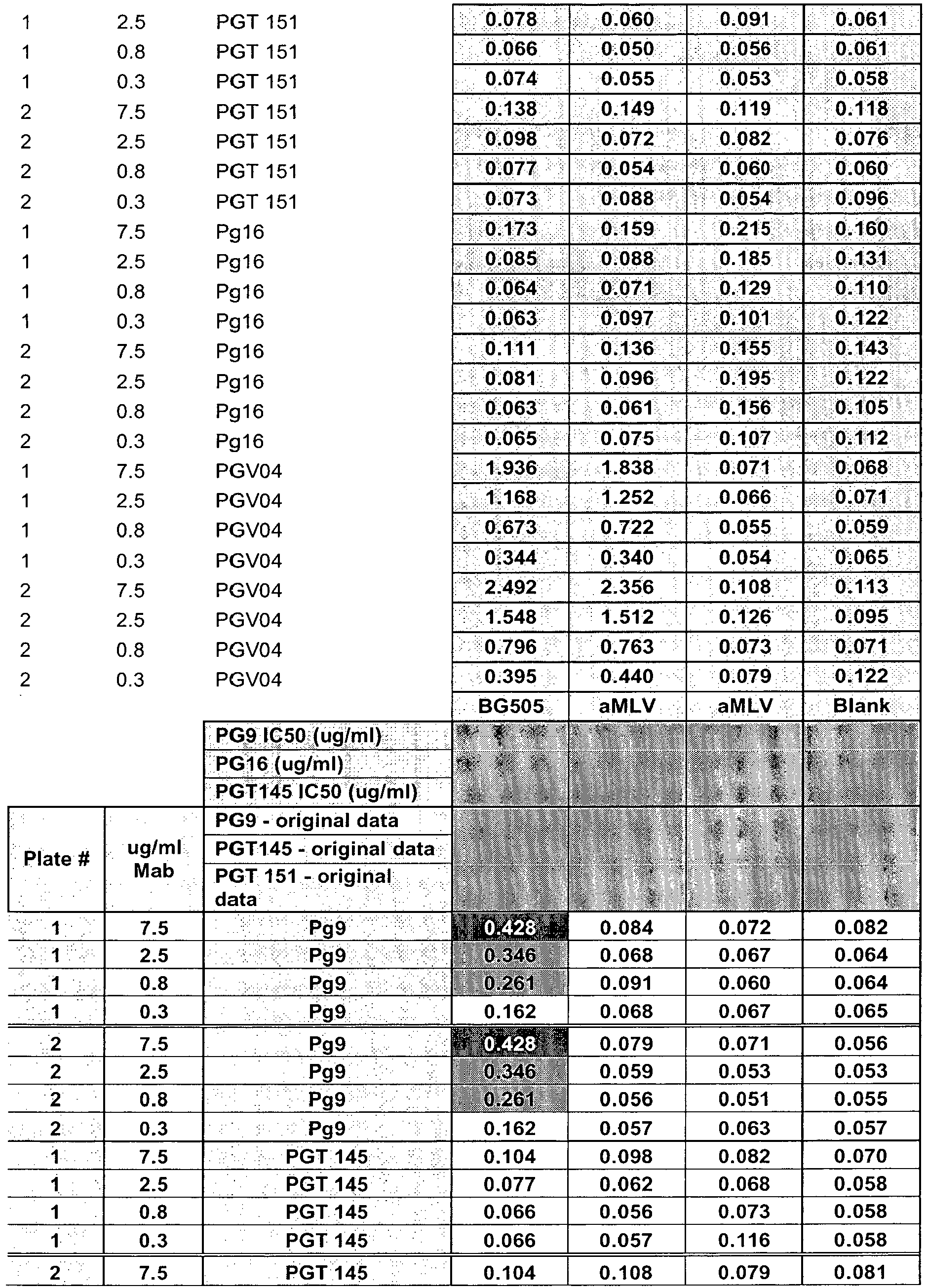

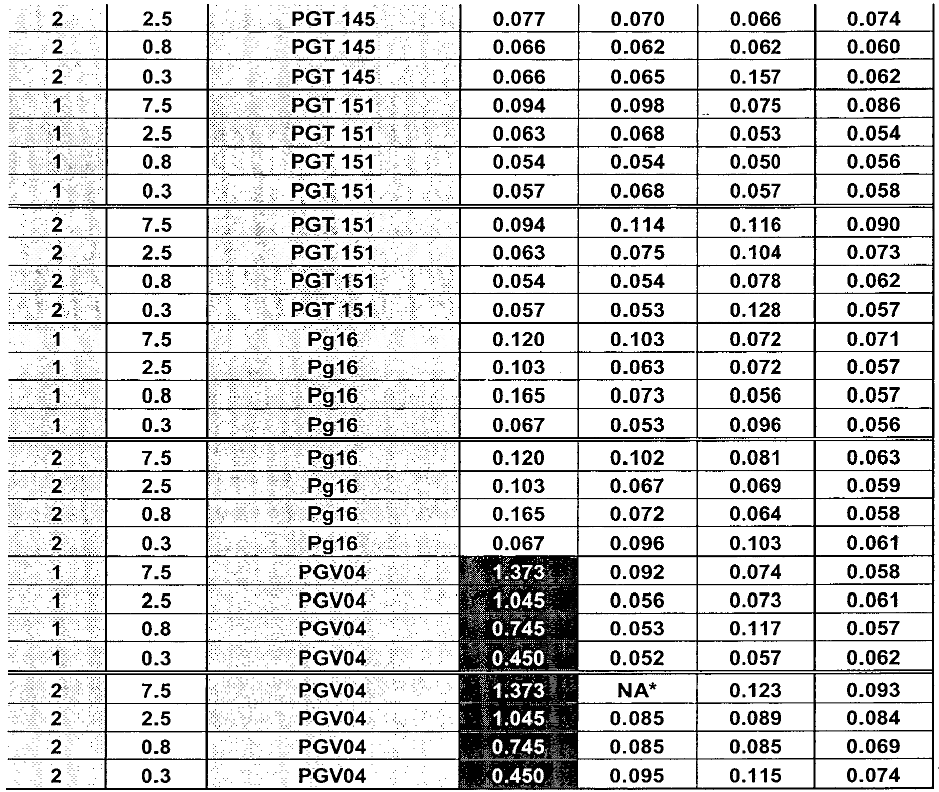

The invention relates to testing pseudoviruses made with clonal envelopes (such as, but not limited to SEQ ID NOS: 1, 2, 3, 4 and/or 5 or variants thereof) in an immunogenic assay, such as ELISA, to measure binding of an envelope component, such as a monomer, to selected monoclonal antibodies (MAbs). The process may include the following steps:

Growth of viral stocks pseudotyped with each of the clonal envelopes.

Lysis of the viral stocks to release gp120 monomers.

Antigen capture ELISA:
(a) An antibody was bound to ELISA plates
(b) Viral supernatant was added to plates.
(c) PGT and PG MAbs were used as primary antibody
(d) Goat anti-human HRP was the secondary antibody

Positive reactions were confirmed at least twice.

Details of the procedure may be found in Development and Characterization of a Novel Single-Cycle Recombinant-Virus Assay To Determine Human Immunodeficiency Virus Type 1 Coreceptor Tropism; Jeannette M. Whitcomb, Wei Huang, Signe Fransen, Kay Limoli, Jonathan Toma, Terri Wrin, Colombe Chappey, Linda D. B. Kiss, Ellen E. Paxinos, and Christos J. Petropoulos; ANTIMICROBIAL AGENTS AND CHEMOTHERAPY, Feb. 2007, p. 566-575 Vol. 51, No. 2 and Development of an HIV-1 Reference Panel of Subtype B Envelope Clones Isolated From the Plasma of Recently Infected Individuals; Becky Schweighardt, Yang Liu, Wei Huang, Colombe Chappey, Yolanda S. Lie, Christos J. Petropoulos, and Terri Wrin; J Acquir Immune Defic Syndr Volume 46, Number 1, September 1, 2007.

The present invention also encompasses epitope mapping. The present invention encompasses mapping regions and/or residues of the envelope that may be bound by neutralizing antibodies. Previously, variation in the genetic sequences of the clones in the population made it impossible to generate clear nucleotide sequences. To perform the mapping, individual clones were selected from the gene population to yield unambiguous sequence. To select individual gene clones, parental plasmid preparation were re-transformed and in E.coli and individual bacterial colonies were selected and expanded, and vector DNA was purified for each clone. Pseudotyped virus stocks were generated for each clone and were characterized for infectivity, cell co-receptor usage (CCR5 and/or CXCR4) and sensitivity to neutralization. Nucleotide sequences were analyzed of gp160 sequences and the neutralization profile of each individual clones was compared to the parental population profile. Clones were selected for inclusion in the based on their similarity to or difference from the parental population and each other. Since the clones are closely related mapping of the amino acid residues important in MAb binding were easier to discern.

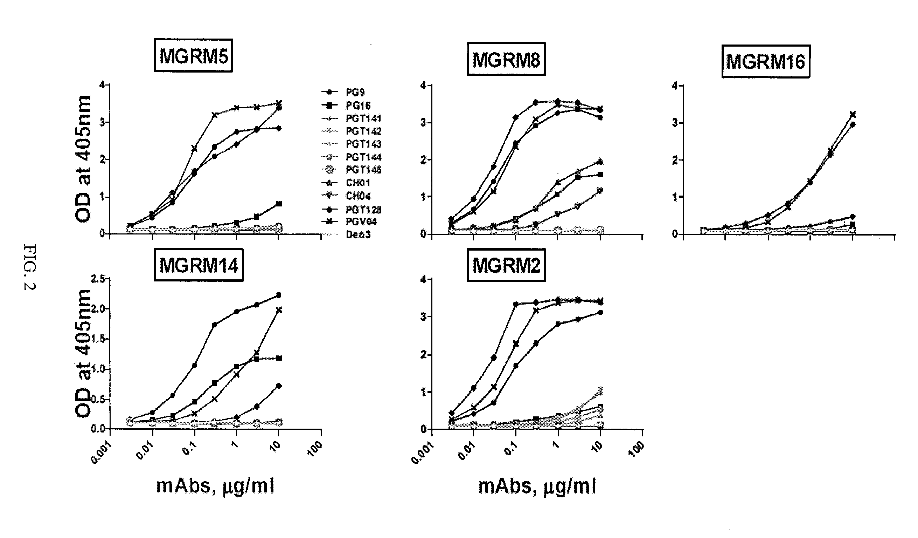

FIG. 2 and Table 2 depict binding of QNE specific mAbs to 293F expressed GNL-SEC purified recombinant gp120s.

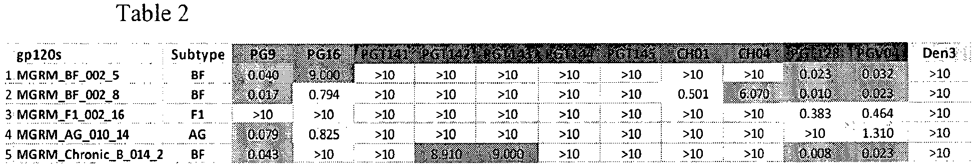

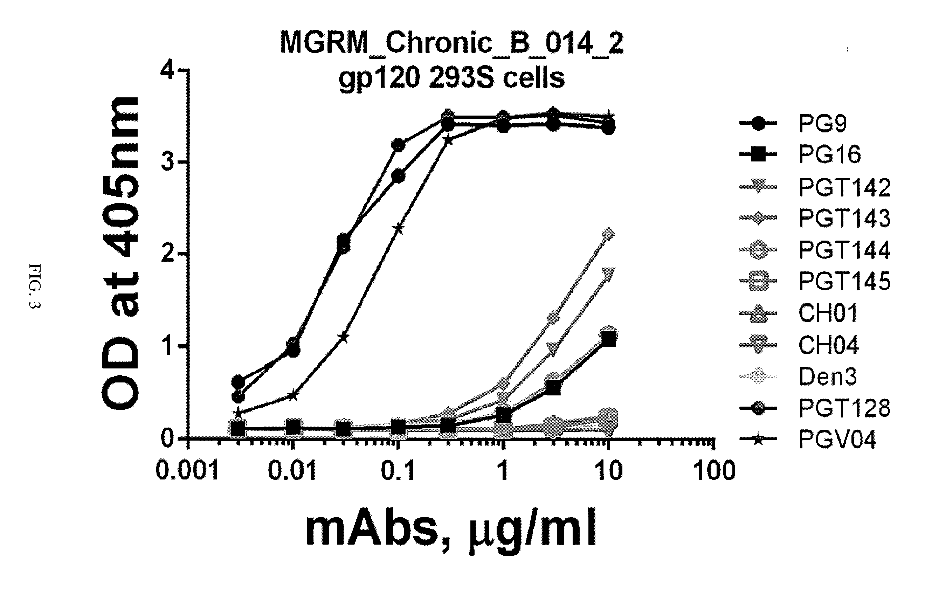

FIG. 3 depicts binding of PGT140 series Abs to GNL MGRM2-gp120 (293S).

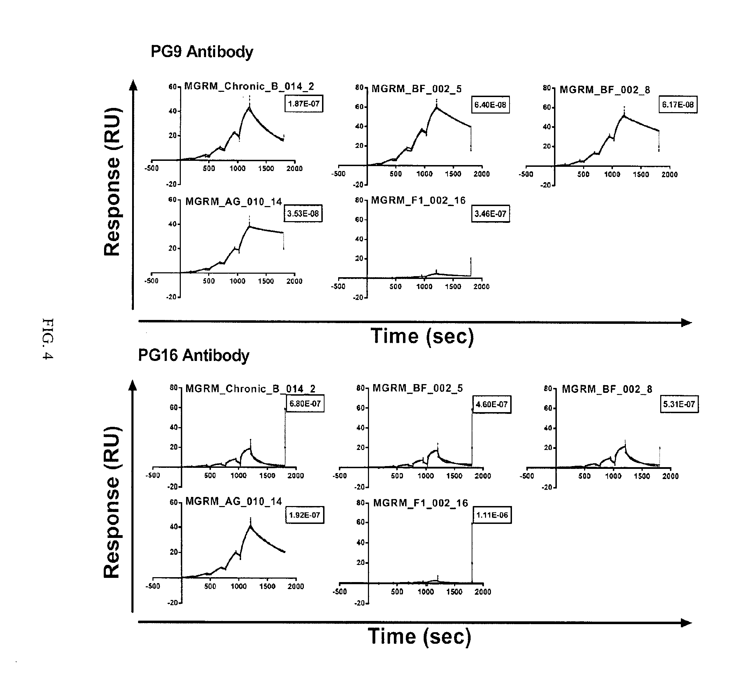

FIG. 4 and Table 3 depict binding affinity of PG9, 16 antibodies to gp120s (SPR).

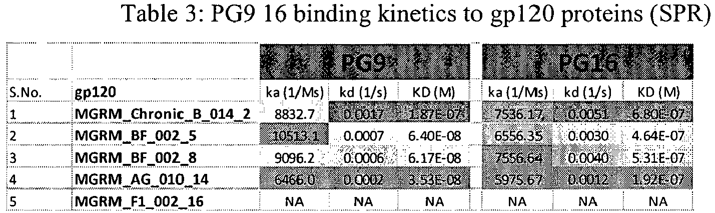

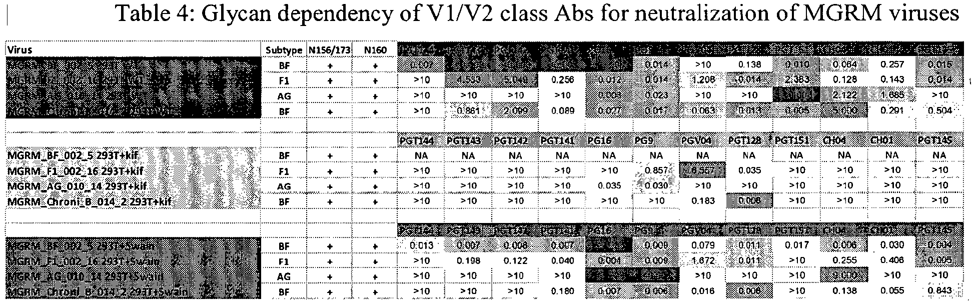

Applicants demonstrate that MGRM8, MGRM14 and MGRM2 bind well to PG9/16 Abs. Applicants further show that MGRM2 shows some reactivity to PGT143. Crystallization is underway with these isolates as gp120s with variable loops or in complex with bNAbs.

In another embodiment of the present invention, the one or more components thereof of an env protein of the present invention may be crystallized in the combination with PGT145, PGT151 or PG9 or with any other neutralizing antibodies, such as PG16 or PGV04, including those identified by the above methods, to determine the exact molecular surface where the soluble envelope glycoprotein binds with the neutralizing antibody to design novel HIV-1 immunogens.

Crystals of the invention may be obtained by conventional means as are well-known in the art of protein crystallography, including batch, liquid bridge, dialysis, vapor diffusion and hanging drop methods (*see, e.g.,* Johnson et al., Biochemistry. 1982 Sep 28;21(20):4839-43; Brayer & McPherson, J Biol Chem. 1982 Apr 10;257(7):3359-61; McPherson &Weickmann, J Biomol Struct Dyn. 1990 Apr;7(5):1053-60; and Koszelak et al., J Mol Biol. 1989 Sep 20;209(2):323-5; Weber et al., Acta Crystallogr B. 1991 Feb 1;47 ( Pt 1):116-27 and Weber, Methods Enzymol. 1991;202:727-41).

Generally, the crystals of the invention are grown by dissolving a substantially pure neutralizing antibody, such as PGT145, PGT151, PG16, PG9 or PGV04, and one or more components thereof of an env protein in an aqueous buffer containing a precipitant at a concentration just below that necessary to precipitate the protein. Water is removed by controlled evaporation to produce precipitating conditions, which are maintained until crystal growth ceases.

The crystals of the invention, and particularly the atomic structure co-ordinates obtained therefrom, have a wide variety of uses. The crystals and structure co-ordinates are particularly useful for identifying compounds that bind to a neutralizing antibody, such as PGT145, PGT151, PG16, PG9 or PGV04, and thus are useful to elicit anti-HIV antibodies. Such compounds may be useful in eliciting clade B and C anti-HIV antibodies, however variants may be useful in eliciting clade A, D or E anti-HIV antibodies.

The structure co-ordinates may be used as phasing models in determining the crystal structures of a synthetic or mutated neutralizing antibody, such as PGT145, PGT151, PG16, PG9 or PGV04, domains, as well as the structures of co-crystals of such domains with ligands.

The provision of the crystal structure of a neutralizing antibody, such as PGT145, PGT151, PG16, PG9 or PGV04, complexed with a soluble envelope glycoprotein provide the skilled artisan with a detailed insight into the mechanisms of action of a neutralizing antibody, such as PGT145, PGT151, PG16, PG9 or PGV04. This insight provides a means to design compounds that bind to a neutralizing antibody, such as PGT145, PGT151, PG16, PG9 or PGV04, and thus to certain anti-HIV antibodies, and therefore compounds that elicit anti-HIV antibodies, which are useful in diagnosis, treatment, or prevention of HIV in an individual in need thereof.

The provision of the crystal structure of a neutralizing antibody, such as PGT145, PGT151, PG16, PG9 or PGV04, complexed with a soluble envelope glycoprotein allows a novel approach for drug or compound discovery, identification, and design for compounds that bind to a neutralizing antibody, such as PGT145, PGT151, PG16, PG9 or PGV04, and thus to anti-HIV antibodies, and therefore compounds that elicit anti-HIV antibodies, which are useful in diagnosis, treatment, or prevention of HIV in an individual in need thereof. Accordingly, the invention provides a computer-based method of rational drug or compound design or identification which comprises: providing the structure of a neutralizing antibody, such as PGT145, PGT151, PG16, PG9 or PGV04, complex as defined by the co-ordinates or the identifying co-ordinates, providing a structure of a candidate compound; and fitting the structure of the candidate to the structure of a neutralizing antibody, such as PGT145, PGT151, PG16, PG9 or PGV04.

In an alternative aspect, the method may use the co-ordinates of atoms of interest of a neutralizing antibody, such as PGT145, PGT151, PG16, PG9 or PGV04, which are in the vicinity of the active site or binding region in order to model the pocket in which the substrate or ligand binds. These co-ordinates may be used to define a space which is then screened *"in silico"* against a candidate molecule. Thus, the invention provides a computer-based method of rational drug or compound design or identification which comprises: providing the co-ordinates of at least selected co-ordinates; providing the structure of a candidate compound; and fitting the structure of the candidate to the selected co-ordinates.

In practice, it may be desirable to model a sufficient number of atoms of a neutralizing antibody, such as PGT145, PGT151, PG16, PG9 or PGV04, as defined by its co-ordinates which represent the active site or binding region. Thus, there can be provided the co-ordinates of at least 5, advantageously at least 10, more advantageously at least 50 and even more advantageously at least 100 atoms of the structure.

Accordingly, the methods of the invention can employ a sub-domain of interest of a neutralizing antibody, such as PGT145, PGT151, PG16, PG9 or PGV04, which is in the vicinity of the active site or binding region, and the invention can provide a computer-based method for identifying or rationally designing a compound or drug which comprises: providing the coordinates of at least a sub-domain of; providing the structure of a candidate modulator or inhibitor of a neutralizing antibody, such as PGT145, PGT151, PG16, PG9 or PGV04; and fitting the structure of the candidate to the co-ordinates of the sub-domain provided.

The invention further provides a method for determining the structure of a binder of a neutralizing antibody, such as PGT145, PGT151, PG16, PG9 or PGV04, bound to a neutralizing antibody, such as PGT145, PGT151, PG16, PG9 or PGV04, comprising: providing a crystal of a neutralizing antibody, such as PGT145, PGT151, PG16, PG9 or PGV04, e.g., according to the invention, soaking the crystal with the binder, and determining the structure of the neutralizing antibody-binder complex. Alternatively or additionally the neutralizing antibody, such as PGT145, PGT151, PG16, PG9 or PGV04, and the binder may be co-crystallized.

The invention also provides a method of analyzing a complex of a neutralizing antibody, such as PGT145, PGT151, PG16, PG9 or PGV04, and a potential binder comprising: employing X-ray crystallographic diffraction data from the complex and a three-dimensional structure of a neutralizing antibody, such as PGT 145, PGT 151, PG16, PG9 or PGV04, or at least a sub-domain thereof, to generate a different Fourier electron density map of the complex; advantageously, the three-dimensional structure being as defined by its atomic co-ordinate data.

Such complexes can be crystallized and analyzed using X-ray diffraction methods, e.g., according to the approaches described by Greer et al., 1994, and difference Fourier electron density maps can be calculated based on X-ray diffraction patterns of soaked or co-crystallized neutralizing antibody, such as PGT145, PGT151, PG16, PG9 or PGV04, and the solved structure of an uncomplexed neutralizing antibody, such as PGT145, PGT151, PG16, PG9 or PGV04. These maps can then be used to determine whether and where a particular potential binder binds to a neutralizing antibody, such as PGT145, PGT151, PG16, PG9 or PGV04, and/or changes the conformation of a neutralizing antibody, such as PGT145, PGT151, PG16, PG9 or PGV04. Electron density maps can be calculated using programs such as those from the CCP4 computer package (Collaborative Computing Project, No. 4. The CCP4 Suite: Programs for Protein Crystallography, Acta Crystallographica, D50, 1994, 760-763). For map visualization and model building programs such as "QUANTA" (1994, San Diego, CA: Molecular Simulations, Jones et al., 1991) can be used.

Determination of the 3D structure of a neutralizing antibody, such as PGT145, PGT151, PG16, PG9 or PGV04, provides important information about the likely active/binding site(s) of a neutralizing antibody, such as PGT145, PGT151, PG16, PG9 or PGV04. This information may be used for rational design of neutralizing antibody binders, e.g., by computational techniques that identify possible binding ligands for the active site(s), by enabling linked-fragment approaches to drug design, and by enabling the identification and location of bound ligands using analyses such as X-ray crystallographic analysis.

In yet another embodiment, the present invention also encompassed the use of the one or more components thereof of an env protein described herein as immunogens, advantageously as HIV-1 vaccine components.

The terms "protein", "peptide", "polypeptide", and "amino acid sequence" are used interchangeably herein to refer to polymers of amino acid residues of any length. The polymer may be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

As used herein, the terms "antigen" or "immunogen" are used interchangeably to refer to a substance, typically a protein, which is capable of inducing an immune response in a subject. The term also refers to proteins that are immunologically active in the sense that once administered to a subject (either directly or by administering to the subject a nucleotide sequence or vector that encodes the protein) is able to evoke an immune response of the humoral and/or cellular type directed against that protein.

The term "antibody" includes intact molecules as well as fragments thereof, such as Fab, F(ab')₂, Fv and scFv which are capable of binding the epitope determinant. These antibody fragments retain some ability to selectively bind with its antigen or receptor and include, for example:

Fab, the fragment which contains a monovalent antigen-binding fragment of an antibody molecule can be produced by digestion of whole antibody with the enzyme papain to yield an intact light chain and a portion of one heavy chain;

Fab', the fragment of an antibody molecule can be obtained by treating whole antibody with pepsin, followed by reduction, to yield an intact light chain and a portion of the heavy chain; two Fab' fragments are obtained per antibody molecule;

F(ab')₂, the fragment of the antibody that can be obtained by treating whole antibody with the enzyme pepsin without subsequent reduction; F(ab')₂ is a dimer of two Fab' fragments held together by two disulfide bonds;

scFv, including a genetically engineered fragment containing the variable region of a heavy and a light chain as a fused single chain molecule.

General methods of making these fragments are known in the art. (See for example, Harlow and Lane, Antibodies: A Laboratory Manual, Cold Spring Harbor Laboratory, New York (1988), which is incorporated herein by reference).

A "neutralizing antibody" may inhibit the entry of HIV-1 virus for example SF162 and/or JRCSF with a neutralization index >1.5 or >2.0. Broad and potent neutralizing antibodies may neutralize greater than about 50% of HIV-1 viruses (from diverse clades and different strains within a clade) in a neutralization assay. The inhibitory concentration of the monoclonal antibody may be less than about 25 mg/ml to neutralize about 50% of the input virus in the neutralization assay.

It should be understood that the proteins, including the antibodies and/or antigens of the invention may differ from the exact sequences illustrated and described herein. Thus, the invention contemplates deletions, additions and substitutions to the sequences shown, so long as the sequences function in accordance with the methods of the invention. In this regard, particularly preferred substitutions will generally be conservative in nature, *i.e.,* those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cysteine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the sequences illustrated and described but possessing minor amino acid substitutions that do not substantially affect the immunogenicity of the protein are, therefore, within the scope of the invention.

As used herein the terms "nucleotide sequences" and "nucleic acid sequences" refer to deoxyribonucleic acid (DNA) or ribonucleic acid (RNA) sequences, including, without limitation, messenger RNA (mRNA), DNA/RNA hybrids, or synthetic nucleic acids. The nucleic acid can be single-stranded, or partially or completely double-stranded (duplex). Duplex nucleic acids can be homoduplex or heteroduplex.

As used herein the term "transgene" may be used to refer to "recombinant" nucleotide sequences that may be derived from any of the nucleotide sequences encoding the proteins of the present invention. The term "recombinant" means a nucleotide sequence that has been manipulated "by man" and which does not occur in nature, or is linked to another nucleotide sequence or found in a different arrangement in nature. It is understood that manipulated "by man" means manipulated by some artificial means, including by use of machines, codon optimization, restriction enzymes, etc.

For example, in one embodiment the nucleotide sequences may be mutated such that the activity of the encoded proteins *in vivo* is abrogated. In another embodiment the nucleotide sequences may be codon optimized, for example the codons may be optimized for human use. In preferred embodiments the nucleotide sequences of the invention are both mutated to abrogate the normal in vivo function of the encoded proteins, and codon optimized for human use. For example, each of the Gag, Pol, Env, Nef, RT, and Int sequences of the invention may be altered in these ways.

As regards codon optimization, the nucleic acid molecules of the invention have a nucleotide sequence that encodes the antigens of the invention and can be designed to employ codons that are used in the genes of the subject in which the antigen is to be produced. Many viruses, including HIV and other lentiviruses, use a large number of rare codons and, by altering these codons to correspond to codons commonly used in the desired subject, enhanced expression of the antigens can be achieved. In a preferred embodiment, the codons used are "humanized" codons, i.e., the codons are those that appear frequently in highly expressed human genes (Andre et al., J. Virol. 72:1497-1503, 1998) instead of those codons that are frequently used by HIV. Such codon usage provides for efficient expression of the transgenic HIV proteins in human cells. Any suitable method of codon optimization may be used. Such methods, and the selection of such methods, are well known to those of skill in the art. In addition, there are several companies that will optimize codons of sequences, such as Geneart (geneart.com). Thus, the nucleotide sequences of the invention can readily be codon optimized.

The invention further encompasses nucleotide sequences encoding functionally and/or antigenically equivalent variants and derivatives of the antigens of the invention and functionally equivalent fragments thereof. These functionally equivalent variants, derivatives, and fragments display the ability to retain antigenic activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the antigen, epitope, immunogen, peptide or polypeptide of interest.

For the purposes of the present invention, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988; 85: 2444-2448.

Advantageous for use according to the present invention is the WU-BLAST (Washington University BLAST) version 2.0 software. WU-BLAST version 2.0 executable programs for several UNIX platforms can be downloaded from ftp://blast.wustl.edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873-5877; all of which are incorporated by reference herein).

The various recombinant nucleotide sequences and antibodies and/or antigens of the invention are made using standard recombinant DNA and cloning techniques. Such techniques are well known to those of skill in the art. See for example, "Molecular Cloning: A Laboratory Manual", second edition (Sambrook et al. 1989).

The nucleotide sequences of the present invention may be inserted into "vectors." The term "vector" is widely used and understood by those of skill in the art, and as used herein the term "vector" is used consistent with its meaning to those of skill in the art. For example, the term "vector" is commonly used by those skilled in the art to refer to a vehicle that allows or facilitates the transfer of nucleic acid molecules from one environment to another or that allows or facilitates the manipulation of a nucleic acid molecule.

Any vector that allows expression of the antibodies and/or antigens of the present invention may be used in accordance with the present invention. In certain embodiments, the antigens and/or antibodies of the present invention may be used *in vitro* (such as using cell-free expression systems) and/or in cultured cells grown *in vitro* in order to produce the encoded HIV-antigens and/or antibodies which may then be used for various applications such as in the production of proteinaceous vaccines. For such applications, any vector that allows expression of the antigens and/or antibodies *in vitro* and/or in cultured cells may be used.

For applications where it is desired that the antibodies and/or antigens be expressed *in vivo,* for example when the transgenes of the invention are used in DNA or DNA-containing vaccines, any vector that allows for the expression of the antibodies and/or antigens of the present invention and is safe for use *in vivo* may be used. In preferred embodiments the vectors used are safe for use in humans, mammals and/or laboratory animals.

For the antibodies and/or antigens of the present invention to be expressed, the protein coding sequence should be "operably linked" to regulatory or nucleic acid control sequences that direct transcription and translation of the protein. As used herein, a coding sequence and a nucleic acid control sequence or promoter are said to be "operably linked" when they are covalently linked in such a way as to place the expression or transcription and/or translation of the coding sequence under the influence or control of the nucleic acid control sequence. The "nucleic acid control sequence" can be any nucleic acid element, such as, but not limited to promoters, enhancers, IRES, introns, and other elements described herein that direct the expression of a nucleic acid sequence or coding sequence that is operably linked thereto. The term "promoter" will be used herein to refer to a group of transcriptional control modules that are clustered around the initiation site for RNA polymerase II and that when operationally linked to the protein coding sequences of the invention lead to the expression of the encoded protein. The expression of the transgenes of the present invention can be under the control of a constitutive promoter or of an inducible promoter, which initiates transcription only when exposed to some particular external stimulus, such as, without limitation, antibiotics such as tetracycline, hormones such as ecdysone, or heavy metals. The promoter can also be specific to a particular cell-type, tissue or organ. Many suitable promoters and enhancers are known in the art, and any such suitable promoter or enhancer may be used for expression of the transgenes of the invention. For example, suitable promoters and/or enhancers can be selected from the Eukaryotic Promoter Database (EPDB).

The present invention relates to a recombinant vector expressing a foreign epitope. Advantageously, the epitope is an HIV epitope. In an advantageous embodiment, the HIV epitope is a soluble envelope glycoprotein, however, the present invention may encompass additional HIV antigens, epitopes or immunogens. Advantageously, the HIV epitope is an HIV antigen, HIV epitope or an HIV immunogen, such as, but not limited to, the HIV antigens, HIV epitopes or HIV immunogens of U.S. Patent Nos. 7,341,731; 7,335,364; 7,329,807; 7,323,553; 7,320,859; 7,311,920; 7,306,798; 7,285,646; 7,285,289; 7,285,271; 7,282,364; 7,273,695; 7,270,997; 7,262,270; 7,244,819; 7,244,575; 7,232,567; 7,232,566; 7,223,844; 7,223,739; 7,223,534; 7,223,368; 7,220,554; 7,214,530; 7,211,659; 7,211,432; 7,205,159; 7,198,934; 7,195,768; 7,192,555; 7,189,826; 7,189,522; 7,186,507; 7,179,645; 7,175,843; 7,172,761; 7,169,550; 7,157,083; 7,153,509; 7,147,862; 7,141,550; 7,129,219; 7,122,188; 7,118,859; 7,118,855; 7,118,751; 7,118,742; 7,105,655; 7,101,552; 7,097,971; 7,097,842; 7,094,405; 7,091,049; 7,090,648; 7,087,377; 7,083,787; 7,070,787; 7,070,781; 7,060,273; 7,056,521; 7,056,519; 7,049,136; 7,048,929; 7,033,593; 7,030,094; 7,022,326; 7,009,037; 7,008,622; 7,001,759; 6,997,863; 6,995,008; 6,979,535; 6,974,574; 6,972,126; 6,969,609; 6,964,769; 6,964,762; 6,958,158; 6,956,059; 6,953,689; 6,951,648; 6,946,075; 6,927,031; 6,919,319; 6,919,318; 6,919,077; 6,913,752; 6,911,315; 6,908,617; 6,908,612; 6,902,743; 6,900,010; 6,893,869; 6,884,785; 6,884,435; 6,875,435; 6,867,005; 6,861,234; 6,855,539; 6,841,381 6,841,345; 6,838,477; 6,821,955; 6,818,392; 6,818,222; 6,815,217; 6,815,201; 6,812,026; 6,812,025; 6,812,024; 6,808,923; 6,806,055; 6,803,231; 6,800,613; 6,800,288; 6,797,811; 6,780,967; 6,780,598; 6,773,920; 6,764,682; 6,761,893; 6,753,015; 6,750,005; 6,737,239; 6,737,067; 6,730,304; 6,720,310; 6,716,823; 6,713,301; 6,713,070; 6,706,859; 6,699,722; 6,699,656; 6,696,291; 6,692,745; 6,670,181; 6,670,115; 6,664,406; 6,657,055; 6,657,050; 6,656,471; 6,653,066; 6,649,409; 6,649,372; 6,645,732; 6,641,816; 6,635,469; 6,613,530; 6,605,427; 6,602,709; 6,602,705; 6,600,023; 6,596,477; 6,596,172; 6,593,103; 6,593,079; 6,579,673; 6,576,758; 6,573,245; 6,573,040; 6,569,418; 6,569,340; 6,562,800; 6,558,961; 6,551,828; 6,551,824; 6,548,275; 6,544,780; 6,544,752; 6,544,728; 6,534,482; 6,534,312; 6,534,064; 6,531,572; 6,531,313; 6,525,179; 6,525,028; 6,524,582; 6,521,449; 6,518,030; 6,518,015; 6,514,691; 6,514,503; 6,511,845; 6,511,812; 6,511,801; 6,509,313; 6,506,384; 6,503,882; 6,495,676; 6,495,526; 6,495,347; 6,492,123; 6,489,131; 6,489,129; 6,482,614; 6,479,286; 6,479,284; 6,465,634; 6,461,615; 6,458,560; 6,458,527; 6,458,370; 6,451,601; 6,451,592; 6,451,323; 6,436,407; 6,432,633; 6,428,970; 6,428,952; 6,428,790; 6,420,139; 6,416,997; 6,410,318; 6,410,028; 6,410,014; 6,407,221; 6,406,710; 6,403,092; 6,399,295; 6,392,013; 6,391,657; 6,384,198; 6,380,170; 6,376,170; 6,372,426; 6,365,187; 6,358,739; 6,355,248; 6,355,247; 6,348,450; 6,342,372; 6,342,228; 6,338,952; 6,337,179; 6,335,183; 6,335,017; 6,331,404; 6,329,202; 6,329,173; 6,328,976; 6,322,964; 6,319,666; 6,319,665; 6,319,500; 6,319,494; 6,316,205; 6,316,003; 6,309,633; 6,306,625; 6,296,807; 6,294,322; 6,291,239; 6,291,157; 6,287,568; 6,284,456; 6,284,194; 6,274,337; 6,270,956; 6,270,769; 6,268,484; 6,265,562; 6,265,149; 6,262,029; 6,261,762; 6,261,571; 6,261,569; 6,258,599; 6,258,358; 6,248,332; 6,245,331; 6,242,461; 6,241,986; 6,235,526; 6,235,466; 6,232,120; 6,228,361; 6,221,579; 6,214,862; 6,214,804; 6,210,963; 6,210,873; 6,207,185; 6,203,974; 6,197,755; 6,197,531; 6,197,496; 6,194,142; 6,190,871; 6,190,666; 6,168,923; 6,156,302; 6,153,408; 6,153,393; 6,153,392; 6,153,378; 6,153,377; 6,146,635; 6,146,614; 6,143,876 6,140,059; 6,140,043; 6,139,746; 6,132,992; 6,124,306; 6,124,132; 6,121,006; 6,120,990; 6,114,507; 6,114,143; 6,110,466; 6,107,020; 6,103,521; 6,100,234; 6,099,848; 6,099,847; 6,096,291; 6,093,405; 6,090,392; 6,087,476; 6,083,903; 6,080,846; 6,080,725; 6,074,650; 6,074,646; 6,070,126; 6,063,905; 6,063,564; 6,060,256; 6,060,064; 6,048,530; 6,045,788; 6,043,347; 6,043,248; 6,042,831; 6,037,165; 6,033,672; 6,030,772; 6,030,770; 6,030,618; 6,025,141; 6,025,125; 6,020,468; 6,019,979; 6,017,543; 6,017,537; 6,015,694; 6,015,661; 6,013,484; 6,013,432; 6,007,838; 6,004,811; 6,004,807; 6,004,763; 5,998,132; 5,993,819; 5,989,806; 5,985,926; 5,985,641; 5,985,545; 5,981,537; 5,981,505; 5,981,170; 5,976,551; 5,972,339; 5,965,371; 5,962,428; 5,962,318; 5,961,979; 5,961,970; 5,958,765; 5,958,422; 5,955,647; 5,955,342; 5,951,986; 5,951,975; 5,942,237; 5,939,277; 5,939,074; 5,935,580; 5,928,930; 5,928,913; 5,928,644; 5,928,642; 5,925,513; 5,922,550; 5,922,325; 5,919,458; 5,916,806; 5,916,563; 5,914,395; 5,914,109; 5,912,338; 5,912,176; 5,912,170; 5,906,936; 5,895,650; 5,891,623; 5,888,726; 5,885,580 5,885,578; 5,879,685; 5,876,731; 5,876,716; 5,874,226; 5,872,012; 5,871,747; 5,869,058; 5,866,694; 5,866,341; 5,866,320; 5,866,319; 5,866,137; 5,861,290; 5,858,740; 5,858,647; 5,858,646; 5,858,369; 5,858,368; 5,858,366; 5,856,185; 5,854,400; 5,853,736; 5,853,725; 5,853,724; 5,852,186; 5,851,829; 5,851,529; 5,849,475; 5,849,288; 5,843,728; 5,843,723; 5,843,640; 5,843,635; 5,840,480; 5,837,510; 5,837,250; 5,837,242; 5,834,599; 5,834,441; 5,834,429; 5,834,256; 5,830,876; 5,830,641; 5,830,475; 5,830,458; 5,830,457; 5,827,749; 5,827,723; 5,824,497; 5,824,304; 5,821,047; 5,817,767; 5,817,754; 5,817,637; 5,817,470; 5,817,318; 5,814,482; 5,807,707; 5,804,604; 5,804,371; 5,800,822; 5,795,955; 5,795,743; 5,795,572; 5,789,388; 5,780,279; 5,780,038; 5,776,703; 5,773,260; 5,770,572; 5,766,844; 5,766,842; 5,766,625; 5,763,574; 5,763,190; 5,762,965; 5,759,769; 5,756,666; 5,753,258; 5,750,373; 5,747,641; 5,747,526; 5,747,028; 5,736,320; 5,736,146; 5,733,760; 5,731,189; 5,728,385; 5,721,095; 5,716,826; 5,716,637; 5,716,613; 5,714,374; 5,709,879; 5,709,860; 5,709,843; 5,705,331; 5,703,057; 5,702,707 5,698,178; 5,688,914; 5,686,078; 5,681,831; 5,679,784; 5,674,984; 5,672,472; 5,667,964; 5,667,783; 5,665,536; 5,665,355; 5,660,990; 5,658,745; 5,658,569; 5,643,756; 5,641,624; 5,639,854; 5,639,598; 5,637,677; 5,637,455; 5,633,234; 5,629,153; 5,627,025; 5,622,705; 5,614,413; 5,610,035; 5,607,831; 5,606,026; 5,601,819; 5,597,688; 5,593,972; 5,591,829; 5,591,823; 5,589,466; 5,587,285; 5,585,254; 5,585,250; 5,580,773; 5,580,739; 5,580,563; 5,573,916; 5,571,667; 5,569,468; 5,558,865; 5,556,745; 5,550,052; 5,543,328; 5,541,100; 5,541,057; 5,534,406; 5,529,765; 5,523,232; 5,516,895; 5,514,541; 5,510,264; 5,500,161; 5,480,967; 5,480,966; 5,470,701; 5,468,606; 5,462,852; 5,459,127; 5,449,601; 5,447,838; 5,447,837; 5,439,809; 5,439,792; 5,418,136; 5,399,501; 5,397,695; 5,391,479; 5,384,240; 5,374,519; 5,374,518; 5,374,516; 5,364,933; 5,359,046; 5,356,772; 5,354,654; 5,344,755; 5,335,673; 5,332,567; 5,320,940; 5,317,009; 5,312,902; 5,304,466; 5,296,347; 5,286,852; 5,268,265; 5,264,356; 5,264,342; 5,260,308; 5,256,767; 5,256,561; 5,252,556; 5,230,998; 5,230,887; 5,227,159; 5,225,347; 5,221,610 5,217,861; 5,208,321; 5,206,136; 5,198,346; 5,185,147; 5,178,865; 5,173,400; 5,173,399; 5,166,050; 5,156,951; 5,135,864; 5,122,446; 5,120,662; 5,103,836; 5,100,777; 5,100,662; 5,093,230; 5,077,284; 5,070,010; 5,068,174; 5,066,782; 5,055,391; 5,043,262; 5,039,604; 5,039,522; 5,030,718; 5,030,555; 5,030,449; 5,019,387; 5,013,556; 5,008,183; 5,004,697; 4,997,772; 4,983,529; 4,983,387; 4,965,069; 4,945,082; 4,921,787; 4,918,166; 4,900,548; 4,888,290; 4,886,742; 4,885,235; 4,870,003; 4,869,903; 4,861,707; 4,853,326; 4,839,288; 4,833,072 and 4,795,739.

In another embodiment, HIV, or immunogenic fragments thereof, may be utilized as the HIV epitope. For example, the HIV nucleotides of U.S. Patent Nos. 7,393,949, 7,374,877, 7,306,901, 7,303,754, 7,173,014, 7,122,180, 7,078,516, 7,022,814, 6,974,866, 6,958,211, 6,949,337, 6,946,254, 6,896,900, 6,887,977, 6,870,045, 6,803,187, 6,794,129, 6,773,915, 6,768,004, 6,706,268, 6,696,291, 6,692,955, 6,656,706, 6,649,409, 6,627,442, 6,610,476, 6,602,705, 6,582,920, 6,557,296, 6,531,587, 6,531,137, 6,500,623, 6,448,078, 6,429,306, 6,420,545, 6,410,013, 6,407,077, 6,395,891, 6,355,789, 6,335,158, 6,323,185, 6,316,183, 6,303,293, 6,300,056, 6,277,561, 6,270,975, 6,261,564, 6,225,045, 6,222,024, 6,194,391, 6,194,142, 6,162,631, 6,114,167, 6,114,109, 6,090,392, 6,060,587, 6,057,102, 6,054,565, 6,043,081, 6,037,165, 6,034,233, 6,033,902, 6,030,769, 6,020,123, 6,015,661, 6,010,895, 6,001,555, 5,985,661, 5,980,900, 5,972,596, 5,939,538, 5,912,338, 5,869,339, 5,866,701, 5,866,694, 5,866,320, 5,866,137, 5,864,027, 5,861,242, 5,858,785, 5,858,651, 5,849,475, 5,843,638, 5,840,480, 5,821,046, 5,801,056, 5,786,177, 5,786,145, 5,773,247, 5,770,703, 5,756,674, 5,741,706, 5,705,612, 5,693,752, 5,688,637, 5,688,511, 5,684,147, 5,665,577, 5,585,263, 5,578,715, 5,571,712, 5,567,603, 5,554,528, 5,545,726, 5,527,895, 5,527,894, 5,223,423, 5,204,259, 5,144,019, 5,051,496 and 4,942,122 are useful for the present invention.

Any epitope recognized by an HIV antibody may be used in the present invention. For example, the anti-HIV antibodies of U.S. Patent Nos. 6,949,337, 6,900,010, 6,821,744, 6,768,004, 6,613,743, 6,534,312, 6,511,830, 6,489,131, 6,242,197, 6,114,143, 6,074,646, 6,063,564, 6,060,254, 5,919,457, 5,916,806, 5,871,732, 5,824,304, 5,773,247, 5,736,320, 5,637,455, 5,587,285, 5,514,541, 5,317,009, 4,983,529, 4,886,742, 4,870,003 and 4,795,739 are useful for the present invention. Furthermore, monoclonal anti-HIV antibodies of U.S. Patent Nos. 7,074,556, 7,074,554, 7,070,787, 7,060,273, 7,045,130, 7,033,593, RE39,057, 7,008,622, 6,984,721, 6,972,126, 6,949,337, 6,946,465, 6,919,077, 6,916,475, 6,911,315, 6,905,680, 6,900,010, 6,825,217, 6,824,975, 6,818,392, 6,815,201, 6,812,026, 6,812,024, 6,797,811, 6,768,004, 6,703,019, 6,689,118, 6,657,050, 6,608,179, 6,600,023, 6,596,497, 6,589,748, 6,569,143, 6,548,275, 6,525,179, 6,524,582, 6,506,384, 6,498,006, 6,489,131, 6,465,173, 6,461,612, 6,458,933, 6,432,633, 6,410,318, 6,406,701, 6,395,275, 6,391,657, 6,391,635, 6,384,198, 6,376,170, 6,372,217, 6,344,545, 6,337,181, 6,329,202, 6,319,665, 6,319,500, 6,316,003, 6,312,931, 6,309,880, 6,296,807, 6,291,239, 6,261,558, 6,248,514, 6,245,331, 6,242,197, 6,241,986, 6,228,361, 6,221,580, 6,190,871, 6,177,253, 6,146,635, 6,146,627, 6,146,614, 6,143,876, 6,132,992, 6,124,132, RE36,866, 6,114,143, 6,103,238, 6,060,254, 6,039,684, 6,030,772, 6,020,468, 6,013,484, 6,008,044, 5,998,132, 5,994,515, 5,993,812, 5,985,545, 5,981,278, 5,958,765, 5,939,277, 5,928,930, 5,922,325, 5,919,457, 5,916,806, 5,914,109, 5,911,989, 5,906,936, 5,889,158, 5,876,716, 5,874,226, 5,872,012, 5,871,732, 5,866,694, 5,854,400, 5,849,583, 5,849,288, 5,840,480, 5,840,305, 5,834,599, 5,831,034, 5,827,723, 5,821,047, 5,817,767, 5,817,458, 5,804,440, 5,795,572, 5,783,670, 5,776,703, 5,773,225, 5,766,944, 5,753,503, 5,750,373, 5,747,641, 5,736,341, 5,731,189, 5,707,814, 5,702,707, 5,698,178, 5,695,927, 5,665,536, 5,658,745, 5,652,138, 5,645,836, 5,635,345, 5,618,922, 5,610,035, 5,607,847, 5,604,092, 5,601,819, 5,597,896, 5,597,688, 5,591,829, 5,558,865, 5,514,541, 5,510,264, 5,478,753, 5,374,518, 5,374,516, 5,344,755, 5,332,567, 5,300,433, 5,296,347, 5,286,852, 5,264,221, 5,260,308, 5,256,561, 5,254,457, 5,230,998, 5,227,159, 5,223,408, 5,217,895, 5,180,660, 5,173,399, 5,169,752, 5,166,050, 5,156,951, 5,140,105, 5,135,864, 5,120,640, 5,108,904, 5,104,790, 5,049,389, 5,030,718, 5,030,555, 5,004,697, 4,983,529, 4,888,290, 4,886,742 and 4,853,326, are also useful for the present invention.

The vectors used in accordance with the present invention should typically be chosen such that they contain a suitable gene regulatory region, such as a promoter or enhancer, such that the antigens and/or antibodies of the invention can be expressed.

For example, when the aim is to express the antibodies and/or antigens of the invention *in vitro,* or in cultured cells, or in any prokaryotic or eukaryotic system for the purpose of producing the protein(s) encoded by that antibody and/or antigen, then any suitable vector can be used depending on the application. For example, plasmids, viral vectors, bacterial vectors, protozoal vectors, insect vectors, baculovirus expression vectors, yeast vectors, mammalian cell vectors, and the like, can be used. Suitable vectors can be selected by the skilled artisan taking into consideration the characteristics of the vector and the requirements for expressing the antibodies and/or antigens under the identified circumstances.

When the aim is to express the antibodies and/or antigens of the invention *in vivo* in a subject, for example in order to generate an immune response against an HIV-1 antigen and/or protective immunity against HIV-1, expression vectors that are suitable for expression on that subject, and that are safe for use *in vivo,* should be chosen. For example, in some embodiments it may be desired to express the antibodies and/or antigens of the invention in a laboratory animal, such as for pre-clinical testing of the HIV-1 immunogenic compositions and vaccines of the invention. In other embodiments, it will be desirable to express the antibodies and/or antigens of the invention in human subjects, such as in clinical trials and for actual clinical use of the immunogenic compositions and vaccine of the invention. Any vectors that are suitable for such uses can be employed, and it is well within the capabilities of the skilled artisan to select a suitable vector. In some embodiments it may be preferred that the vectors used for these *in vivo* applications are attenuated to vector from amplifying in the subject. For example, if plasmid vectors are used, preferably they will lack an origin of replication that functions in the subject so as to enhance safety for *in vivo* use in the subject.. If viral vectors are used, preferably they are attenuated or replication-defective in the subject, again, so as to enhance safety for *in vivo* use in the subject.

In preferred embodiments of the present invention viral vectors are used. Viral expression vectors are well known to those skilled in the art and include, for example, viruses such as adenoviruses, adeno-associated viruses (AAV), alphaviruses, herpesviruses, retroviruses and poxviruses, including avipox viruses, attenuated poxviruses, vaccinia viruses, and particularly, the modified vaccinia Ankara virus (MVA; ATCC Accession No. VR-1566). Such viruses, when used as expression vectors are innately non-pathogenic in the selected subjects such as humans or have been modified to render them non-pathogenic in the selected subjects. For example, replication-defective adenoviruses and alphaviruses are well known and can be used as gene delivery vectors.

The nucleotide sequences and vectors of the invention can be delivered to cells, for example if aim is to express and the HIV-1 antigens in cells in order to produce and isolate the expressed proteins, such as from cells grown in culture. For expressing the antibodies and/or antigens in cells any suitable transfection, transformation, or gene delivery methods can be used. Such methods are well known by those skilled in the art, and one of skill in the art would readily be able to select a suitable method depending on the nature of the nucleotide sequences, vectors, and cell types used. For example, transfection, transformation, microinjection, infection, electroporation, lipofection, or liposome-mediated delivery could be used. Expression of the antibodies and/or antigens can be carried out in any suitable type of host cells, such as bacterial cells, yeast, insect cells, and mammalian cells. The antibodies and/or antigens of the invention can also be expressed using including *in vitro* transcription/translation systems. All of such methods are well known by those skilled in the art, and one of skill in the art would readily be able to select a suitable method depending on the nature of the nucleotide sequences, vectors, and cell types used.

In preferred embodiments, the nucleotide sequences, antibodies and/or antigens of the invention are administered *in vivo,* for example where the aim is to produce an immunogenic response in a subject. A "subject" in the context of the present invention may be any animal. For example, in some embodiments it may be desired to express the transgenes of the invention in a laboratory animal, such as for pre-clinical testing of the HIV-1 immunogenic compositions and vaccines of the invention. In other embodiments, it will be desirable to express the antibodies and/or antigens of the invention in human subjects, such as in clinical trials and for actual clinical use of the immunogenic compositions and vaccine of the invention. In preferred embodiments the subject is a human, for example a human that is infected with, or is at risk of infection with, HIV-1.

For such *in vivo* applications the nucleotide sequences, antibodies and/or antigens of the invention are preferably administered as a component of an immunogenic composition comprising the nucleotide sequences and/or antigens of the invention in admixture with a pharmaceutically acceptable carrier. The immunogenic compositions of the invention are useful to stimulate an immune response against HIV-1 and may be used as one or more components of a prophylactic or therapeutic vaccine against HIV-1 for the prevention, amelioration or treatment of AIDS. The nucleic acids and vectors of the invention are particularly useful for providing genetic vaccines, i.e. vaccines for delivering the nucleic acids encoding the antibodies and/or antigens of the invention to a subject, such as a human, such that the antibodies and/or antigens are then expressed in the subject to elicit an immune response.

The compositions of the invention may be injectable suspensions, solutions, sprays, lyophilized powders, syrups, elixirs and the like. Any suitable form of composition may be used. To prepare such a composition, a nucleic acid or vector of the invention, having the desired degree of purity, is mixed with one or more pharmaceutically acceptable carriers and/or excipients. The carriers and excipients must be "acceptable" in the sense of being compatible with the other ingredients of the composition. Acceptable carriers, excipients, or stabilizers are nontoxic to recipients at the dosages and concentrations employed, and include, but are not limited to, water, saline, phosphate buffered saline, dextrose, glycerol, ethanol, or combinations thereof, buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

An immunogenic or immunological composition can also be formulated in the form of an oil-in-water emulsion. The oil-in-water emulsion can be based, for example, on light liquid paraffin oil (European Pharmacopea type); isoprenoid oil such as squalane, squalene, EICOSANE™ or tetratetracontane; oil resulting from the oligomerization of alkene(s), e.g., isobutene or decene; esters of acids or of alcohols containing a linear alkyl group, such as plant oils, ethyl oleate, propylene glycol di(caprylate/caprate), glyceryl tri(caprylate/caprate) or propylene glycol dioleate; esters of branched fatty acids or alcohols, e.g., isostearic acid esters. The oil advantageously is used in combination with emulsifiers to form the emulsion. The emulsifiers can be nonionic surfactants, such as esters of sorbitan, mannide (e.g., anhydromannitol oleate), glycerol, polyglycerol, propylene glycol, and oleic, isostearic, ricinoleic, or hydroxystearic acid, which are optionally ethoxylated, and polyoxypropylene-polyoxyethylene copolymer blocks, such as the Pluronic® products, *e.g.,* L121. The adjuvant can be a mixture of emulsifier(s), micelle-forming agent, and oil such as that which is commercially available under the name Provax® (IDEC Pharmaceuticals, San Diego, CA).

The immunogenic compositions of the invention can contain additional substances, such as wetting or emulsifying agents, buffering agents, or adjuvants to enhance the effectiveness of the vaccines (Remington's Pharmaceutical Sciences, 18th edition, Mack Publishing Company, (ed.) 1980).

Adjuvants may also be included. Adjuvants include, but are not limited to, mineral salts (e.g., AlK(SO₄)₂, AlNa(SO₄)₂, AlNH(SO₄)₂, silica, alum, Al(OH)₃, Ca₃(PO₄)₂, kaolin, or carbon), polynucleotides with or without immune stimulating complexes (ISCOMs) (e.g., CpG oligonucleotides, such as those described in Chuang, T.H. et al, (2002) J. Leuk. Biol. 71(3): 538-44; Ahmad-Nejad, P. et al (2002) Eur. J. Immunol. 32(7): 1958-68; poly IC or poly AU acids, polyarginine with or without CpG (also known in the art as IC31; see Schellack, C. et al (2003) Proceedings of the 34th Annual Meeting of the German Society of Immunology; Lingnau, K. et al (2002) Vaccine 20(29-30): 3498-508), JuvaVax™ (U.S. Patent No. 6,693,086), certain natural substances (e.g., wax D from *Mycobacterium tuberculosis,* substances found in *Cornyebacterium parvum, Bordetella pertussis,* or members of the genus *Brucella*)*,* flagellin (Toll-like receptor 5 ligand; see McSorley, S.J. et al (2002) J. Immunol. 169(7): 3914-9), saponins such as QS21, QS17, and QS7 (U.S. Patent Nos. 5,057,540; 5,650,398; 6,524,584; 6,645,495), monophosphoryl lipid A, in particular, 3-de-O-acylated monophosphoryl lipid A (3D-MPL), imiquimod (also known in the art as IQM and commercially available as Aldara®; U.S. Patent Nos. 4,689,338; 5,238,944; Zuber, A.K. et al (2004) 22(13-14): 1791-8), and the CCR5 inhibitor CMPD167 (see Veazey, R.S. et al (2003) J. Exp. Med. 198: 1551-1562).

Aluminum hydroxide or phosphate (alum) are commonly used at 0.05 to 0.1% solution in phosphate buffered saline. Other adjuvants that can be used, especially with DNA vaccines, are cholera toxin, especially CTA1-DD/ISCOMs (see Mowat, A.M. et al (2001) J. Immunol. 167(6): 3398-405), polyphosphazenes (Allcock, H.R. (1998) App. Organometallic Chem. 12(10-11): 659-666; Payne, L.G. et al (1995) Pharm. Biotechnol. 6: 473-93), cytokines such as, but not limited to, IL-2, IL-4, GM-CSF, IL-12, IL-15 IGF-1, IFN-α, IFN-β, and IFN-γ (Boyer et al., (2002) J. Liposome Res. 121:137-142; WO01/095919), immunoregulatory proteins such as CD40L (ADX40; see, for example, WO03/063899), and the CD1a ligand of natural killer cells (also known as CRONY or α-galactosyl ceramide; see Green, T.D. et al, (2003) J. Virol. 77(3): 2046-2055), immunostimulatory fusion proteins such as IL-2 fused to the Fc fragment of immunoglobulins (Barouch et al., Science 290:486-492, 2000) and co-stimulatory molecules B7.1 and B7.2 (Boyer), all of which can be administered either as proteins or in the form of DNA, on the same expression vectors as those encoding the antigens of the invention or on separate expression vectors.

In an advantageous embodiment, the adjuvants may be lecithin combined with an acrylic polymer (Adjuplex-LAP), lecithin coated oil droplets in an oil-in-water emulsion (Adjuplex-LE) or lecithin and acrylic polymer in an oil-in-water emulsion (Adjuplex-LAO) (Advanced BioAdjuvants (ABA)).

The immunogenic compositions can be designed to introduce the nucleic acids or expression vectors to a desired site of action and release it at an appropriate and controllable rate. Methods of preparing controlled-release formulations are known in the art. For example, controlled release preparations can be produced by the use of polymers to complex or absorb the immunogen and/or immunogenic composition. A controlled-release formulation can be prepared using appropriate macromolecules (for example, polyesters, polyamino acids, polyvinyl, pyrrolidone, ethylenevinylacetate, methylcellulose, carboxymethylcellulose, or protamine sulfate) known to provide the desired controlled release characteristics or release profile. Another possible method to control the duration of action by a controlled-release preparation is to incorporate the active ingredients into particles of a polymeric material such as, for example, polyesters, polyamino acids, hydrogels, polylactic acid, polyglycolic acid, copolymers of these acids, or ethylene vinylacetate copolymers. Alternatively, instead of incorporating these active ingredients into polymeric particles, it is possible to entrap these materials into microcapsules prepared, for example, by coacervation techniques or by interfacial polymerization, for example, hydroxymethylcellulose or gelatin-microcapsule and poly-(methylmethacrylate) microcapsule, respectively, in colloidal drug delivery systems (for example, liposomes, albumin microspheres, microemulsions, nano-particles and nanocapsules) or in macroemulsions. Such techniques are disclosed in New Trends and Developments in Vaccines, Voller et al. (eds.), University Park Press, Baltimore, Md., 1978 and Remington's Pharmaceutical Sciences, 16th edition.

Suitable dosages of the nucleic acids and expression vectors of the invention (collectively, the immunogens) in the immunogenic composition of the invention can be readily determined by those of skill in the art. For example, the dosage of the immunogens can vary depending on the route of administration and the size of the subject. Suitable doses can be determined by those of skill in the art, for example by measuring the immune response of a subject, such as a laboratory animal, using conventional immunological techniques, and adjusting the dosages as appropriate. Such techniques for measuring the immune response of the subject include but are not limited to, chromium release assays, tetramer binding assays, IFN-γ ELISPOT assays, IL-2 ELISPOT assays, intracellular cytokine assays, and other immunological detection assays, e.g., as detailed in the text "Antibodies: A Laboratory Manual" by Ed Harlow and David Lane.

When provided prophylactically, the immunogenic compositions of the invention are ideally administered to a subject in advance of HIV infection, or evidence of HIV infection, or in advance of any symptom due to AIDS, especially in high-risk subjects. The prophylactic administration of the immunogenic compositions can serve to provide protective immunity of a subject against HIV-1 infection or to prevent or attenuate the progression of AIDS in a subject already infected with HIV-1. When provided therapeutically, the immunogenic compositions can serve to ameliorate and treat AIDS symptoms and are advantageously used as soon after infection as possible, preferably before appearance of any symptoms of AIDS but may also be used at (or after) the onset of the disease symptoms.

The immunogenic compositions can be administered using any suitable delivery method including, but not limited to, intramuscular, intravenous, intradermal, mucosal, and topical delivery. Such techniques are well known to those of skill in the art. More specific examples of delivery methods are intramuscular injection, intradermal injection, and subcutaneous injection. However, delivery need not be limited to injection methods. Further, delivery of DNA to animal tissue has been achieved by cationic liposomes (Watanabe et al., (1994) Mol. Reprod. Dev. 38:268-274; and WO 96/20013), direct injection of naked DNA into animal muscle tissue (Robinson et al., (1993) Vaccine 11:957-960; Hoffman et al., (1994) Vaccine 12: 1529-1533; Xiang et al., (1994) Virology 199: 132-140; Webster et al., (1994) Vaccine 12: 1495-1498; Davis et al., (1994) Vaccine 12: 1503-1509; and Davis et al., (1993) Hum. Mol. Gen. 2: 1847-1851), or intradermal injection of DNA using "gene gun" technology (Johnston et al., (1994) Meth. Cell Biol. 43:353-365). Alternatively, delivery routes can be oral, intranasal or by any other suitable route. Delivery also be accomplished via a mucosal surface such as the anal, vaginal or oral mucosa.

Immunization schedules (or regimens) are well known for animals (including humans) and can be readily determined for the particular subject and immunogenic composition. Hence, the immunogens can be administered one or more times to the subject. Preferably, there is a set time interval between separate administrations of the immunogenic composition. While this interval varies for every subject, typically it ranges from 10 days to several weeks, and is often 2, 4, 6 or 8 weeks. For humans, the interval is typically from 2 to 6 weeks. The immunization regimes typically have from 1 to 6 administrations of the immunogenic composition, but may have as few as one or two or four. The methods of inducing an immune response can also include administration of an adjuvant with the immunogens. In some instances, annual, biannual or other long interval (5-10 years) booster immunization can supplement the initial immunization protocol.

The present methods also include a variety of prime-boost regimens, for example DNA prime-Adenovirus boost regimens. In these methods, one or more priming immunizations are followed by one or more boosting immunizations. The actual immunogenic composition can be the same or different for each immunization and the type of immunogenic composition (e.g., containing protein or expression vector), the route, and formulation of the immunogens can also be varied. For example, if an expression vector is used for the priming and boosting steps, it can either be of the same or different type (e.g., DNA or bacterial or viral expression vector). One useful prime-boost regimen provides for two priming immunizations, four weeks apart, followed by two boosting immunizations at 4 and 8 weeks after the last priming immunization. It should also be readily apparent to one of skill in the art that there are several permutations and combinations that are encompassed using the DNA, bacterial and viral expression vectors of the invention to provide priming and boosting regimens.

A specific embodiment of the invention provides methods of inducing an immune response against HIV in a subject by administering an immunogenic composition of the invention, preferably comprising an adenovirus vector containing DNA encoding one or more of the epitopes of the invention, one or more times to a subject wherein the epitopes are expressed at a level sufficient to induce a specific immune response in the subject. Such immunizations can be repeated multiple times at time intervals of at least 2, 4 or 6 weeks (or more) in accordance with a desired immunization regime.

The immunogenic compositions of the invention can be administered alone, or can be co-administered, or sequentially administered, with other HIV immunogens and/or HIV immunogenic compositions, e.g., with "other" immunological, antigenic or vaccine or therapeutic compositions thereby providing multivalent or "cocktail" or combination compositions of the invention and methods of employing them. Again, the ingredients and manner (sequential or co-administration) of administration, as well as dosages can be determined taking into consideration such factors as the age, sex, weight, species and condition of the particular subject, and the route of administration.

When used in combination, the other HIV immunogens can be administered at the same time or at different times as part of an overall immunization regime, e.g., as part of a prime-boost regimen or other immunization protocol. In an advantageous embodiment, the other HIV immunogen is env, preferably the HIV env trimer.

Many other HIV immunogens are known in the art, one such preferred immunogen is HIVA (described in WO 01/47955), which can be administered as a protein, on a plasmid (e.g., pTHr.HIVA) or in a viral vector (e.g., MVA.HIVA). Another such HIV immunogen is RENTA (described in PCT/US2004/037699), which can also be administered as a protein, on a plasmid (e.g., pTHr.RENTA) or in a viral vector (e.g., MVA.RENTA).

For example, one method of inducing an immune response against HIV in a human subject comprises administering at least one priming dose of an HIV immunogen and at least one boosting dose of an HIV immunogen, wherein the immunogen in each dose can be the same or different, provided that at least one of the immunogens is an epitope of the present invention, a nucleic acid encoding an epitope of the invention or an expression vector, preferably a VSV vector, encoding an epitope of the invention, and wherein the immunogens are administered in an amount or expressed at a level sufficient to induce an HIV-specific immune response in the subject. The HIV-specific immune response can include an HIV-specific T-cell immune response or an HIV-specific B-cell immune response. Such immunizations can be done at intervals, preferably of at least 2-6 or more weeks.

Although the present invention and its advantages have been described in detail, it should be understood that various changes, substitutions and alterations can be made herein without departing from the spirit and scope of the invention as defined in the appended claims.

The invention is further described by the following numbered paragraphs:
1. An isolated or non-naturally occurring HIV-1 envelope glycoprotein encoded by SEQ ID NO: 1, SEQ ID NO: 2, SEQ ID NO: 3, SEQ ID NO: 4, or SEQ ID NO. 5.
2. A vector comprising an isolated or non-naturally occurring nucleic acid comprising SEQ ID NO: 1, SEQ ID NO: 2, SEQ ID NO: 3, SEQ ID NO: 4, or SEQ ID NO. 5.
3. A host cell comprising the vector of paragraph 2.
4. A method of eliciting an immune response comprising administering to a mammal a composition comprising the glycoprotein of paragraph 1.
5. The method of paragraph 4, wherein the composition further comprises an adjuvant.
6. The method of paragraph 5, wherein the adjuvant comprises a lecithin.
7. The method of paragraph 6, wherein the adjuvant is a lecithin combined with an acrylic polymer, a lecithin coated oil droplet in an oil-in-water emulsion, or a lecithin and an acrylic polymer in an oil-in-water emulsion.
8. A method of eliciting an immune response comprising administering to a mammal a composition comprising an isolated or non-naturally occurring nucleic acid comprising SEQ ID NO: 1, SEQ ID NO: 2, SEQ ID NO: 3, SEQ ID NO: 4, or SEQ ID NO. 5.
9. A method of eliciting an immune response comprising administering to a mammal a composition comprising the vector of paragraph 2.
10. A method of eliciting an immune response comprising administering to a mammal a composition comprising the host cell of paragraph 3.
11. An immunogenic composition comprising the isolated HIV-1 envelope glycoprotein of paragraph 1.
12. The immunogenic composition of paragraph 11, wherein the isolated HIV-1 envelope glycoprotein is present as a monomer.
13. The immunogenic composition of paragraph 11, wherein the isolated HIV-1 envelope glycoprotein is present as a trimer.
14. A method of eliciting an immune response in a mammal comprising administering to the mammal the immunogenic composition of paragraph 11.
15. A method for identifying a viral envelope polypeptide monomer which binds to a broadly neutralizing antibody, comprising:
   a) isolating a nucleic acid sequence encoding an envelope polypeptide from a patient or parental viral population,
   b) generating pseudovirus stocks comprising the envelope polypeptide from the patient or parental viral population,
   c) characterizing the phenotype of the pseudovirus stocks,
   d) sequencing the nucleic acid sequence encoding the envelope polypeptides, and
   e) identifying clones based on similarity to or difference from the parental population and other clones, thereby identifying a viral envelope polypeptide monomer which binds to the broadly neutralizing antibody.
16. The method of paragraph 15, wherein the broadly neutralizing antibody is PGT145, PGT151, or PG9.
17. The method of paragraph 15, wherein the characterizing step comprises determining the infectivity of the pseudovirus stock, determining the cell co-receptor usage of the pseudovirus stock, determining the sensitivity of the pseudovirus stock to neutralization by a monoclonal antibody (MAb) or polyclonal HIV+ plasma or sera, or a combination thereof.
18. The method of paragraph 15, wherein the characterizing step is performed in the presence or absence of a disrupting agent.
19. The method of paragraph 18, wherein the disrupting agent is a detergent.
20. The method of paragraph 15, wherein the characterizing step comprises a binding assay.
21. The method of paragraph 20, wherein the binding assay is an ELISA.
22. The method of paragraph 15, wherein the viral envelope polypeptide monomer is encoded by SEQ ID NO: 1, SEQ ID NO: 2, SEQ ID NO: 3, SEQ ID NO: 4, or SEQ ID NO. 5.
23. A method of mapping regions or residues of an envelope polypeptide monomer bound by a neutralizing antibody, comprising the steps of the method of paragraph 15 and further comprising:
   f) identifying the amino acids important in the binding of the envelope polypeptide monomer to the antibody in closely related clones.
24. The method of paragraph 23, wherein the broadly neutralizing antibody is PGT145, PGT151, /or PG9.
25. The method of paragraph 23, wherein the characterizing step comprises determining the infectivity of the pseudovirus stock, determining the cell co-receptor usage of the pseudovirus stock, and determining the sensitivity of the pseudovirus stock to neutralization by a monoclonal antibody (MAb) or polyclonal HIV+ plasma or sera.
26. The method of paragraph 23, wherein the characterizing step is performed in the presence or absence of a disrupting agent.
27. The method of paragraph 26, wherein the disrupting agent is a detergent.
28. The method of paragraph 23, wherein the characterizing step comprises a binding assay.
29. The method of paragraph 28, wherein the binding assay is an ELISA.
30. The method of paragraph 23, wherein the viral envelope polypeptide monomer is encoded by SEQ ID NO: 1, SEQ ID NO: 2, SEQ ID NO: 3, SEQ ID NO: 4, or SEQ ID NO. 5.
31. A method for identifying a component of HIV envelope glycoprotein (env) which bind to broadly neutralizing antibodies comprising cloning and purifying a viral env gene from a parental population, generating pseudotype stocks comprising the viral env gene, propagating the pseudovirus stocks, attaching soluble proteins from the pseudovirus stocks to a solid surface and detecting of binding to the broadly neutralizing antibodies, thereby identifying a component of env which bind to broadly neutralizing antibodies.
32. The method of paragraph 31, wherein the detecting of binding is with an antigen capture ELISA.
33. The method of paragraph 31 or 32, wherein the broadly neutralizing antibodies are PG9, PG16, PGT145, PGT151 and/or PGV04.
34. The method of any one of paragraphs 31-33, wherein the viral envelope gene comprises a nucleic acid encoded by SEQ ID NO: 1, SEQ ID NO: 2, SEQ ID NO: 3, SEQ ID NO: 4 or SEQ ID NO. 5.
35. A method of mapping regions and/or residues of an envelope bound by neutralizing antibodies comprising the method of any one of paragraphs 31-34 and further comprising nucleotide sequence analysis of all gp160 sequences, and identifying amino acids important in monoclonal antibody binding in closely related clones.
36. The method of paragraph 35, wherein the characterization of pseudotype stocks comprises infectivity, cell co-receptor usage (CCR5 and/or CXCR4) and sensitivity to neutralization by a panel of monoclonal antibodies (MAb) and/or polyclonal HIV+ plasma/sera.

Having thus described in detail preferred embodiments of the present invention, it is to be understood that the invention defined by the above paragraphs is not to be limited to particular details set forth in the above description as many apparent variations thereof are possible without departing from the spirit or scope of the present invention.

## Claims

1. A method for identifying a component of HIV envelope glycoprotein (env) which bind to broadly neutralizing antibodies comprising cloning and purifying a viral envelope gene from a parental population, generating pseudotype stocks comprising the viral envelope gene, propagating the pseudovirus stocks, attaching soluble proteins from the pseudovirus stocks to a solid surface and detecting of binding to the broadly neutralizing antibodies, thereby identifying a component of env which bind to broadly neutralizing antibodies.

2. The method of claim 1, wherein the detecting of binding is with an antigen capture ELISA.

3. The method of claim 1 or 2, wherein the broadly neutralizing antibodies are PG9, PG16, PGT 145, PGT151 and/or PGV04.

4. The method of any of claims 1-3, wherein the viral envelope gene comprises a nucleic acid encoded by SEQ ID NO: 1, SEQ ID NO: 2, SEQ ID NO: 3, SEQ ID NO: 4 or SEQ ID NO. 5.

5. A method of mapping regions and/or residues of an envelope bound by neutralizing antibodies comprising the method of any of claims 1-4 and further comprising nucleotide sequence analysis of all gp160 sequences, and identifying amino acids important in monoclonal antibody binding in closely related clones.

6. The method of claim 5, wherein the characterization of pseudotype stocks comprises infectivity, cell co-receptor usage (CCR5 and/or CXCR4) and sensitivity to neutralization by a panel of monoclonal antibodies (MAb) and/or polyclonal HIV+ plasma/sera.
